# EUROPEAN PATENT SPECIFICATION

(11) **EP 2 214 677 B1**
(45) Date of publication and mention of the grant of the patent: **30.08.2017**
(21) Application number: 08848917.4
(22) Date of filing: 12.11.2008
(51) Int. Cl.: A61K 9/00, A61K 9/06, A61K 47/36, A61K 31/41, A61K 31/573, A61K 47/38

(54) **COMPOSITIONS FOR THE TREATMENT OF INFLAMMATION OF THE GASTROINTESTINAL TRACT**
ZUSAMMENSETZUNGEN ZUR BEHANDLUNG VON ENTZÜNDUNGEN DES MAGEN-DARM-TRAKTS
COMPOSITIONS POUR LE TRAITEMENT DE L'INFLAMMATION DES VOIES GASTRO-INTESTINALES

(30) Priority: 13.11.2007 US 987720 P; 06.12.2007 US 12012; 21.12.2007 US 15998; 08.01.2008 US 19818; 07.03.2008 US 34941; 10.03.2008 US 35348; 16.05.2008 US 54103; 16.05.2008 US 54104; 16.05.2008 US 54105; 16.05.2008 US 54106; 16.05.2008 US 54107; 20.08.2008 US 90568
(43) Date of publication of application: 11.08.2010
(73) Proprietor: Meritage Pharma, Inc., Lexington, MA 02421 (US)
(72) Inventor: HILL, Malcolm, San Diego, California 92130 (US)
(74) Representative: Goodfellow, Hugh Robin
(86) International application number: PCT/US2008/012781
(87) International publication number: WO 2009/064458

(56) References cited:
- WO-A1-2005/074930
- WO-A2-01/41748
- WO-A2-2006/103702
- US-A1- 2001 049 366
- US-A1- 2007 111 978
- US-A1- 2007 111 978
- US-B1- 6 387 383
- CARO, J.J. ET AL.: 'HEALING AND RELAPSE RATES IN GASTROESOPHAGEAL REFLUX DISEASE TREATED WITH THE NEWER PROTON-PUMP INHIBITORS LANSOPRAZOLE, RABEPRAZOLE, AND PANTOPRAZOLE COMPARED WITH OMEPRAZOLE, RANITIDINE, AND PLACEBO: EVIDENCE FROM RANDOMIZED CLINICAL TRIALS' CLINICAL THERAPEUTICS vol. 23, no. 7, 2001, pages 998 - 1017, XP008133514
- ANDERSEN ET AL: "Treatment of esophageal strictures with intralesional steroids", GASTROINTESTINAL ENDOSCOPY, ELSEVIER, NL, vol. 41, no. 4, 1 April 1995 (1995-04-01), page 333, XP005452239, ISSN: 0016-5107, DOI: 10.1016/S0016-5107(05)80186-5

## Description

### BACKGROUND OF THE INVENTION

Gastroesophageal reflux disease (GERD) is among the most common gastrointestinal (GI) problems. GERD is caused by abnormal reflux in the esophagus. Heartburn is a common symptom that is indicative of GERD. Other symptoms associated with GERD include, by way of non-limiting example, odynophagia, bitter taste in the mouth, belching, nausea, dysphagia, regurgitation, laryngitis, cough, hoarseness and asthma are also associated with GERD. Andersen et al disclose the treatment of esophageal strictures associated with GERD ("Treatment of esophageal strictures with intralesional steroids", GASTROINTESTINAL ENDOSCOPY, vol. 41, no. 4, 1995, page 333)

### SUMMARY OF THE INVENTION

The application relates to a composition comprising a therapeutically effective amount of topical corticosteroid for use in a method of treating or alleviating the symptoms of or inflammation associated with gastroesophageal reflux disease (GERD) in an individual, wherein the topical corticosteroid is budesonide, fluticasone, mometasone furoate, ciclesonide, beclomethasone, desonide, or a pharmaceutically acceptable ester thereof, or a combination thereof, by topical administration to the esophagus of the individual. Accordingly, certain embodiments of the present invention provide for a method of treating or alleviating the symptoms of or inflammation associated with gastroesophageal reflux disease (GERD). Specifically, some embodiments of the present invention provide for a method of treating or alleviating the symptoms of or inflammation associated with gastroesophageal reflux disease (GERD) in an individual by administering to an individual a therapeutically effective amount of a corticosteroid. In specific embodiments, the gastroesophageal reflux disease treated nonerosive reflux disease (NERD). In other specific embodiments, the gastroesophageal reflux disease is erosive esophagitis (EE). The corticosteroid utilized in the methods described herein is a topical corticosteroid. Specific topical corticosteroid include, by way of non-limiting example, budesonide and fluticasone.

In certain embodiments, the methods described herein include administration of about 0.1 mg to about 20 mg/day; or at least 250 µg/day of the corticosteroid to the individual. In specific embodiments, between about 300 µg/day and about 4 mg/day, or between about 500 µg/day and about 6 mg/day of the corticosteroid is administered to the individual. In more specific embodiments, between about 500 µg/day and about 3 mg/day of the corticosteroid is administered to the individual. In some embodiments, less than 500 µg/day of the corticosteroid is administered to the individual.

In some embodiments, the methods described herein further include administering a therapeutically effective amount of an acid inhibitor to the individual.

In certain embodiments, the acid inhibitor is an H₂RA. In some embodiments, the corticosteroid and H₂RA are administered concurrently. In specific embodiments, the H₂RA is selected from, by way of non-limiting example, cimetidine, famotidine, nizatidine, and ranitidine. In more specific embodiments, the H₂RA is ranitidine. In some embodiments, the H₂RA is administered in an amount of between 1 mg and 500 mg.

In other embodiments, the acid inhibitor is a proton pump inhibitor. In some embodiments, the corticosteroid and the proton pump inhibitor are administered concurrently. In specific embodiments, the proton pump inhibitor is selected from, by way of non-limiting example, omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, S-tenatoprazole-Na, and dexlansoprazole. In a more specific embodiment, the proton pump inhibitor is omeprazole. In certain embodiments, the proton pump inhibitor is administered in an amount of between 1 mg and 600 mg. Furthermore, in addition to administering therapeutically effective amounts of a corticosteroid and a proton pump inhibitor, certain embodiments of the present invention include methods further comprising administering a therapeutically effective amount of an H₂RA to said individual.

In any of the methods described herein, the present invention includes methods wherein the corticosteroid is administered in the form of a pharmaceutical composition comprising the corticosteroid and at least one excipient. In specific embodiments, such a pharmaceutical composition is viscous. In other embodiments, the pharmaceutical composition is non-viscous. In some embodiments, the excipient increases the interaction of the composition with the individual's esophagus. In certain embodiments, the excipient is a viscosity enhancer, a mucoadhesive agent, an absorption enhancing agent, or a combination thereof. As used herein, a mucoadhesive agent is an agent that adheres to a gastrointestinal surface (e.g., either or both of a gastrointestinal epithelia or mucosa).

In some embodiments, the viscosity-enhancing excipient is selected from, by way of non-limiting example, cellulose (including cellulose derivatives), acacia (gum arabic), agar, aluminum magnesium silicate, sodium alginate, sodium stearate, bladderwrack, bentonite, carbomer, carrageenan, Carbopol, cellulose, microcrystalline cellulose (MCC), ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), Splenda® (distributed by McNeil Nutritionals, LLC Fort Washington, PA 19034-2299; comprising dextrose, maltodextrin and sucralose) and combinations thereof. In specific embodiments, the viscosity-enhancing excipient is a combination of MCC and CMC (e.g., Avicel RC-591).

In some embodiments, the viscosity of the pharmaceutical composition is greater than about 2 mPa.s, greater than about 50 mPa.s, about 50 mPa.s to about 800 mPa.s, or about 90 mPa.s to about 200 mPa.s, or about 300 mPa.s to about 800 mPa.s, or about 300 mPa.s to about 500 mPa.s or about 400 mPa.s to about 600 mPa.s, and wherein the viscosity is measured at 25 degrees Celsius. In specific embodiments, the viscosity of the pharmaceutical composition is about 250 mPa.s to about 600 mPa.s.

In certain embodiments, the mucoadhesive agent is selected from, by way of non-limiting example, a soluble polyvinylpyrrolidone polymer (PVP); a water-swellable, but water-insoluble, fibrous, cross-linked carboxy-functional polymer, a cross-linked poly(acrylic acid), a carbomer homopolymer, a carbomer copolymer, a hydrophilic polysaccharide gum, maltodextrin, a cross-linked alignate gum gel, a water-dispersible polycarboxylated vinyl polymer, and combinations thereof. In other embodiments, the mucoadhesive agent is selected from at least of titanium dioxide, silicon dioxide, and clay, and mixtures thereof.

In some embodiments, the absorption enhancing agent is selected from, by way of non-limiting example, acylcarnitines, surfactants, sodium lauryl sulfate, saponins, bile salts or bile acids including but not limited to cholanic acid, chilic acid, deoxycholic acid, glycocholic acid, tautocholic acid, chenodeoxycholic acid, lithocholic acid, ursocholic acid, ursodeoxycholic acid, isourosde oxycholic acid, lagodeoxycholic acid, glycodeoxycholic acid, glycochenodeoxycholic acid, dehydrocholic acid, hyocholic acid, hyodeoxycholic acid, or combinations thereof, dihydrofusidates, fatty acid derivatives, chitosan, carbopol, cellulosic agents, sterols, including but not limited to alcohols structurally related to steroids, including but not limited to cholestanol, coprostanol, cholesterol, epicholesterol, ergosterol, ergocalciferol, or combinations thereof, starch, dextran, cyclodextrin, and combinations thereof.

In certain embodiments, the present invention provides for methods wherein the corticosteroid is administered in a unit dose formulation for oral administration.

In some embodiments, the individual is an adult. In other embodiments, the individual is a child or infant. In certain embodiments, the child or infant is less than 19 years old, less than 12 years old, less than 8 years old, less than 6 years old, less than 4 years old or less than 2 years old.

### DETAILED DESCRIPTION OF THE INVENTION

Provided herein are compositions and methods for the treatment of symptoms and/or inflammation associated with gastroesophageal reflux disease (GERD). In some embodiments provided herein are methods for treating the symptoms of and/or inflammation associated with the gastrointestinal tract (e.g., associated with reflux disorders of the gastrointestinal tract) in an individual comprising orally administering to said individual a corticosteroid. In some embodiments provided herein are methods for treating the symptoms of and/or inflammation associated with gastroesophageal reflux disease (GERD) in an individual comprising orally administering to said individual a corticosteroid. In some embodiments, the symptom of GERD treated is heartburn, acid regurgitation, acid sensitivity, chest pain and/or feeding intolerance.

Although most incidences of acid reflux are not sensed, GERD symptoms occur in about 50% of patients at least once a month and in about 20% of patients at least once a week. Patients with GERD score lower in quality of life assessments than do patients with congestive heart failure or even cardiac angina. Billions of dollars are spent in the United States for the treatment of GERD each year.

While transient reflux episodes may occur naturally, GERD is associated with the impairment of normal esophageal defenses. In GERD patients, gastroesophageal reflux may cause the display of inflammation of the squamous mucosa of the esophagus and/or hyperplasia of the epithelium. Inflammation resulting from GERD can be found in the absence of *Helicobacter pylori* and other causes of gastritis. In GERD, histologic changes to the squamous mucosa are most likely to occur close to the squamo-columnar junction (Z-line). Traditional reactive changes in the squamous mucosa are typically found at least 3 cm above the Z-line.

Nonerosive reflux disease (NERD) and erosive esophagitis (EE) are the main presentations of gastroesophageal reflux disease (GERD), with NERD being the most common. Despite suffering from a nonerosive form of GERD, NERD patients suffer from similar or identical symptoms as those associated with EE. Furthermore, NERD patients suffer from these symptoms with the same severity and are experience the same level of impairment to their quality of life. In some instances, patients with NERD actually suffer from more severe heartburn symptoms than EE patients. Barret's Esophagus is a more rare and severe form of erosive GERD.

Approximately 50% of NERD patients display excess acid reflux and an additional 50% appear to have esophageal acid exposure within the normal physiological range. There are several theories why these patients, particularly those with acid exposure within the normal physiological range, experience GERD symptoms. Some patients may be highly sensitive to physiological amounts of acid exposure, some patients may experience GERD symptoms as a result of nonacid related esophageal stimuli, and some patients may be sensitive to changes in esophageal pH. Unlike NERD, about 75-90% of EE patients are exposed to excess acid in the esophagus. Furthermore, the increased exposure to the excess acid in the esophagus is associated with the erosive characteristic of EE. Finally, GERD patients suffering from Barret's Esophagus appear to be exposed to the highest level of esophageal acid when compared to patients suffering from either NERD or EE.

In some embodiments, the present invention provides for methods of treating the symptoms of and/or inflammation associated with nonerosive reflux disease (NERD). In other embodiments, the present invention provides methods of preventing the symptoms of and/or inflammation associated with erosive esophagitis (EE). In still other embodiments, the present invention provides for methods of treating the symptoms of and/or inflammation associated with Barret's Esophagus. In some embodiments, the present invention provides methods of treating neutrophilic inflammation associated with GERD. In certain embodiments, the present invention provides for a method of treating an individual suffering from symptoms of and/or inflammation associated with GERD, wherein the individual is not concurrently suffering from eosinophilic esophagitis. In certain embodiments, the present invention provides for a method of treating an individual suffering from symptoms of and/or inflammation associated with GERD, wherein the individual has <15 eosinophils/HPF. In certain embodiments, provided herein is a method of treating non-eosinophilic esophagitis or gastritis by administering to an individual a therapeutically effective amount of corticosteroid, e.g., in a composition as described herein.

In some embodiments, an individual treated according to a method described herein is diagnosed with, displaying the symptoms of, or suspected of having GERD and eosinophilic esophagitis (EoE). In some embodiments, an individual treated according to a method described herein is diagnosed with, displaying the symptoms of, or suspected of having GERD has an eosinophil count of greater than 0, but less than 7 or less than 15 eosinophils/HPF. In certain embodiments, provided herein is a method of treating gastroesophageal reflux disease (GERD) in an individual by administering to the individual a therapeutically effective amount of a corticosteroid, wherein the GERD is refractory (e.g., non-responsive or substantially non-responsive) to at least one acid inhibitor (e.g., at least one PPI and/or H₂RA).

In some embodiments, the present invention provides for methods of treating the symptoms of and/or inflammation associated with gastroesophageal reflux disease (GERD) in an individual by administering to the individual a therapeutically effective amount of a corticosteroid and a therapeutically effective amount of a second agent. In certain embodiments, the second agent is an acid inhibitor. In some embodiments, the corticosteroid and the second agent are administered in combination. In other embodiments, the corticosteroid and the second agent are administered sequentially.

As used herein, unless otherwise stated, the use of the terms "a" and "the" include both singular and multiple embodiments.

As used herein, the phrase "method of treating" or "method for treating" encompasses methods of preventing, reducing the incidences of, providing prophylactic treatment, treating and alleviating.

As used herein, the term "or" includes "and" and "or".

As used herein, the phrase "treating GERD" includes treating symptoms of GERD and treating inflammation associated with GERD.

As used herein, the phrase "a therapeutically effective amount" is an amount sufficient to elicit a change in the symptoms of or inflammation associated with GERD, or other conditions as appropriate within the context of the use of this term.

### Compounds

Compounds useful in the present invention include topical steroids that may be used to treat GERD, including erosive esophagitis, non-erosive reflux disease, and/or Barrett's Esophagus. In one embodiment, the topical steroid is budesonide. In another embodiment, the topical steroid is fluticasone or fluticasone propionate. In certain embodiments, the compounds useful herein are corticosteroids.

Corticosteroids useful in any of the methods and/or pharmaceutical compositions disclosed herein include beclometasone, budesonide, ciclesonide, desonide, fluticasone, mometasone furoate, and combinations, pharmaceutically acceptable esters thereof. In one embodiment of the present invention, the corticosteroid used herein is budesonide. Budesonide is also known as 16,17-(butylidenebis(oxy))-11,21-dihydroxy-, (11-β,16-α)-pregna-1,4-diene-3,20-dione. In another specific embodiment, the corticosteroid is fluticasone or fluticasone propionate. As used herein, any reference to a topical steroid or a corticosteroid, includes the disclosure of a pharmaceutically acceptable salt thereof.

In certain embodiments, the corticosteroid(s) utilized herein are utilized as particles (e.g., corticosteroid particles suspended or dispersed in an aqueous medium). In specific embodiments, the particles are microparticles. In some embodiments, the microparticles have a mean diameter of about 0.1 microns to about 50 microns. In specific embodiments, the microparticles have a mean diameter of about 1 micron to about 20 microns. In certain embodiments, at least 95%, at least 98%, or at least 99% of the microparticles have a diameter of less than 10 microns.

In some embodiments, a composition or formulation described herein comprises less than 50% w/w, less than 40% w/w, less than 30% w/w, less than 20% w/w, less than 10% w/w, less than 8% w/w, less than 6% w/w, less than 5% w/w, less than 4% w/w, less than 3% w/w, less than 2% w/w, or about 2% w/w, less than 1% w/w, less than 0.5% w/w, less than 0.3% w/w, less than 0.2% w/w, or about 0.2% w/w of undissolved particles. In certain embodiments, a composition or formulation described herein is substantially free of non-corticosteroid particles.

Furthermore, any suitable additional active agent for treating GERD is optionally included in a composition or method described herein. In specific embodiments, a composition or formulation described herein comprises a therapeutically effective amount of a corticosteroid and a therapeutically effective amount of at least one additional active agent. In some embodiments, the at least one additional active agent is an agent that treats, prevents, or alleviates the symptoms of and/or inflammation associated with inflammatory diseases involving the gastrointestinal tract (e.g., esophagus). It is to be understood that in certain instances, when the corticosteroid is combined with an additional active agent, the therapeutically effective amount of the corticosteroid is less than it when the additional active agent is absent.

In certain embodiments, the present invention provides for methods and pharmaceutical compositions for preventing, reducing the incidence of, treating or alleviating the symptoms of and inflammation associated with gastroesophageal reflux disease (GERD) in an individual by administering a therapeutically effective amount of a corticosteroid and a therapeutically effective amount of an additional active agent (e.g., an acid inhibitor). In some embodiments, the therapeutically effective amount of the corticosteroid is less when the corticosteroid therapy is administered with (either concurrent with or separate from) an additional active agent (e.g., an acid inhibitor) useful for treating GERD than when administered without the additional active agent (e.g., an acid inhibitor). Furthermore, in various embodiments, the therapeutically effective amount of the additional active agent (e.g., an acid inhibitor) is less than would have been required if administered without the corticosteroid.

Furthermore, provided herein are methods of treating, preventing or alleviating GERD in an individual comprising orally administering to the individual a corticosteroid in association or combination with at least one additional active agent. In certain embodiments, the corticosteroid and the at least one additional active agent is in a single dosage form. In other embodiments, the corticosteroid and the at least one additional active agent are in separate dosage forms and are administered in any manner, including, by way of non-limiting example, simultaneously, sequentially, or at different times. For example, in certain embodiments, several doses of a corticosteroid composition are administered over a period of time, after which administration of the corticosteroid composition is discontinued and administration of at least one additional active agent is administered at least once.

In some embodiments, the at least one additional active agent utilized in a composition, formulation or method described herein is an agent that treats, prevents, or alleviates the symptoms of and/or inflammation associated with GERD. In more specific embodiments, the at least one additional active agent is not a second corticosteroid. In certain embodiments, the at least one additional active agent is an acid inhibitor (e.g., an H2 antagonist and/or a PPI). In certain embodiments, the at least one additional active agent is, by way of non-limiting example, a proton pump inhibitor (PPI), a H2 antagonist, a transient lower esophageal sphincter relaxation (TLESR)-reducing agent, a serotonergic agent/prokinetics, a potassium-competitive acid blocker (P-CAB), a mucosal protectant, a histamine H3 agonist, an anti-gastrin agent, mGluR₅ antagonists, acetylcholine modulator, 5HT₄ receptor agonist, 5HT₃ receptor antagonist, 5HT₁ receptor antagonist, or combinations thereof.

In some embodiments, the acid inhibitor is a proton pump inhibitor (PPI). In certain embodiments, the corticosteroid and the PPI are administered in combination. In some embodiments, the present invention provides for pharmaceutical compositions for treating GERD that comprise a therapeutically effective amount of a corticosteroid and a therapeutically effective amount of a PPI. In some embodiments, the PPI may be coated with a protective layer, for example, an enteric coating, to protect against an acidic environment, such as the stomach, for later delivery at a target area, such as the lower gastrointestinal tract, including the duodenum.

PPIs useful herein include, by way of non-limiting example, omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, S-tenatoprazole-Na, and dexlansoprazole.

In various embodiments, the acid inhibitor is a histamine-2 or H₂ receptor antagonist (H₂RA) or H₂ blocker. In certain embodiments, the corticosteroid and the H₂RA are administered in combination. In some embodiments, the present invention provides for pharmaceutical compositions for treating GERD that comprise a therapeutically effective amount of a corticosteroid and a therapeutically effective amount of an H₂RA. As used herein, use of the term "H₂RA" includes disclosure related to both H₂ receptor antagonists and/or H₂ blockers.

H₂RAs useful herein include, by way of non-limiting example, cimetidine, rantitidine, famotidine and nizatidine.

In some embodiments, the TLESR-reducing agent is selected from, by way of non-limiting example, GABA_{B} agonists (e.g., baclofen), cholecystokinin (CCK-A or CCK-1) antagonists, anticholinergic agents, NO synthase inhibitors and combinations thereof. In some embodiments, the serotonergic agent/prokinetic is a 5-HT₄ receptor agonist (e.g., a selective 5-HT₄ receptor agonist) including, by way of non-limiting example, cisapride, mosapride, tegaserod, ATI-7505 and combinations thereof. In some embodiments, potassium competitive acid blocker (P-CAB) is selected from, by way of non-limiting example, soraprazan (BY359), revaprazan (YH1885), AZD0865, CS-526 and combinations thereof. In certain embodiments, mucosal protectants are selected from, by way of non-limiting example, sucralfate. In some embodiments, mucosal protectants include one or more of prostaglandin E₂ (PGE₂), epidermal growth factor (EGF) and/or transforming growth factor-α (TGF- α), or analogs thereof. In a specific embodiment, the mucosal protectant comprises the PGE₂ analog trimoprostil. In some embodiments, the histamine H3 agonist is selected from, by way of non-limiting example, (R)-α-methyl-histamine. In certain embodiments, the anti-gastrin agent is selected from, by way of non-limiting example, cholecystokinin (CCK-B or CCK-2) antagonists. Cholecystokinin (CCK-B or CCK-2) antagonists include, by way of non-limiting example, Z-360.

In various embodiments, the corticosteroid described herein is combined with at least one excipient. In some embodiments, the excipient may increase the interaction of the composition with a surface (e.g., a mucosal or epithelial layer) of the gastrointestinal tract (e.g., esophagus), including excipients that increase the viscosity of the composition, impart a mucoadhesive characteristic to the composition, or enhance the absorption of the composition through a surface (e.g., a mucosal or epithelial layer) of the gastrointestinal tract (e.g., esophagus).

In certain embodiments, the excipient or excipients chosen increase the interaction of the composition with the surface of the gastrointestinal tract (e.g., the mucosa and/or epithelium of the gastrointestinal tract or of a specific site of the gastrointestinal tract, such as the esophagus) by at least 1.02 fold, by at least 1.05-fold, by at least 1.1 fold, by at least 1.2 fold, by at least 1.25-fold, by at least 1.5-fold, by at least 2-fold, by at least 3-fold, by at least 4-fold or by at least 5-fold. In certain embodiments, the increased interaction of the composition is an at least 1.02 fold, by at least 1.05-fold, by at least 1.1 fold, by at least 1.2 fold, by at least 1.25-fold, by at least 1.5-fold, by at least 2-fold, by at least 3-fold, by at least 4-fold or by at least 5-fold of interaction of the composition with the esophagus that occurs following passing of the bolus of the composition being swallowed. In certain embodiments, these increases are measured and compared to the measure of an otherwise similar composition lacking the excipient or excipients that increase the interaction of the composition with the surface of the gastrointestinal tract. In certain instances, increased interaction of the composition is measured as a function of the amount of composition present in the esophagus (e.g., after the bolus has passed through the esophagus). In specific instances, the amount of composition present in the esophagus is measured in any suitable manner, e.g., by radiolabeling the composition and measuring the amount of the composition in the esophagus utilizing gamma scintigraphy. An increase in the interaction of the composition with the mucosal layer may be measured by a variety of means. In one example, the retention time of the material along a length of a mucosal layer may be measured, wherein the retention time is increased in the presence of the excipients as compared to its absence. In another aspect, the amount of composition absorbed may also be quantified as a measurement of the interaction of the composition with a mucosal layer. In yet another aspect, the residence time of the composition may also be quantified, with the residence time increased in the presence of the excipients as compared to its absence. In another embodiment, uptake measurements of the active agents may be compared in the presence and absence of excipients, wherein the rate or amount of uptake may indicate an increase in the interaction of the compositions disclosed herein with a surface (e.g., a mucosal or epithelial layer) of the gastrointestinal tract (e.g., esophagus). In yet another embodiment, an increased interaction may be measured by the decrease in physiological manifestations or symptoms of the disease or ailment to be treated. In other embodiments, changes in permeability or other cellular characteristics of a surface (e.g., a mucosal or epithelial layer) of the gastrointestinal tract (e.g., esophagus) may also be quantified, wherein a change in permeability may indicate an increase in the interaction of the compositions disclosed herein with a surface (e.g., a mucosal or epithelial layer) of the gastrointestinal tract (e.g., esophagus).

In one aspect of the invention, the use of the excipients may act to decrease the quantity of active agents needed to elicit a response in the absence of the excipients. In some embodiments, the excipients may decrease the amount of corticosteroid used. Similarly, the excipients may decrease the amount of acid inhibitor needed, for example, from about 1 mg to about 750 mg acid inhibitor in the absence of excipient, to about 500 ug to about 600 mg acid inhibitor in the presence of excipient.

Provided herein are methods and pharmaceutical compositions for treating the symptoms of and/or inflammation associated with gastroesophageal reflux disease (GERD), including erosive esophagitis (EE), non-erosive reflux disease (NERD) and Barrett's Esophagus.

In one aspect, provided herein is an oral pharmaceutical composition comprising (i) a corticosteroid and (ii) an H₂RA.

In another aspect, provided herein is an oral pharmaceutical composition for treating the symptoms of and/or inflammation associated with GERD comprising (i) a corticosteroid; (ii) an H₂RA; and (iii) an excipient or combination of excipients. In some embodiments, the excipient may increase the interaction of the composition with a surface (e.g., a mucosal or epithelial layer) of the gastrointestinal tract (e.g., esophagus), including excipients that increase the viscosity of the composition, impart a mucoadhesive characteristic to the composition, or enhance the absorption of the composition through a surface (e.g., a mucosal or epithelial layer) of the gastrointestinal tract (e.g., esophagus).

In one aspect, provided herein is an oral pharmaceutical composition for treating the symptoms of and/or inflammation associated with GERD comprising (i) a corticosteroid and (ii) a proton pump inhibitor (PPI).

In another aspect, provided herein is an oral pharmaceutical composition for treating the symptoms of and/or inflammation associated with GERD comprising (i) a corticosteroid; (ii) a proton pump inhibitor (PPI); and (iii) an excipient or combination of excipients. In some embodiments, the excipient may increase the interaction of the composition with a surface (e.g., a mucosal or epithelial layer) of the gastrointestinal tract (e.g., esophagus), including excipients that increase the viscosity of the composition, impart a mucoadhesive characteristic to the composition, or enhance the absorption of the composition through a surface (e.g., a mucosal or epithelial layer) of the gastrointestinal tract (e.g., esophagus). In some embodiments, the PPI may be coated with a protective layer, for example, an enteric coating, to protect against an acidic environment, such as the stomach, for later delivery at a target area, such as the lower gastrointestinal tract, including the duodenum.

In one aspect, provided herein is an oral pharmaceutical composition for treating the symptoms of and/or inflammation associated with GERD comprising (i) a corticosteroid, (ii) a PPI, (iii) an H₂RA and (iv) an excipient or combination of excipients thereof. In some embodiments, the excipient may increase the interaction of the composition with a surface (e.g., a mucosal or epithelial layer) of the gastrointestinal tract (e.g., esophagus), including excipients that increase the viscosity of the composition, impart a mucoadhesive characteristic to the composition, or enhance the absorption of the composition through a surface (e.g., a mucosal or epithelial layer) of the gastrointestinal tract (e.g., esophagus). In some embodiments, the PPI may be coated with a protective layer, for example, an enteric coating, to protect against an acidic environment, such as the stomach, for later delivery at a target area, such as the lower gastrointestinal tract, including the duodenum.

In certain embodiments, pharmaceutical compositions disclosed herein and used herein comprise one or more excipients and/or one or more additional active agents. Excipients useful herein include, by way of non-limiting example, mucoadhesive agents, viscosity enhancing agents, binders, fillers, lubricants, solvents, suspension agents, flavoring agents, coloring agents, sweeteners, preservatives, antioxidants, buffering agents, humectants, chelating agents, surfactants, and the like. Additional active agents useful herein include, by way of non-limiting example, a proton pump inhibitor (PPI), a H2 antagonist, a transient lower esophageal sphincter relaxation (TLESR)-reducing agent, a serotonergic agent/prokinetics, a potassium-competitive acid blocker (P-CAB), a mucosal protectant, a histamine H3 agonist, an anti-gastrin agent, mGluR₅ antagonists, acetylcholine modulator, 5HT₄ receptor agonist, 5HT₃ receptor antagonist, 5HT₁ receptor antagonist, antibiotics, or combinations thereof. In certain instances, an additional active agent useful herein also serves to extend the time of contact between the composition and a surface (e.g., a mucosal or epithelial layer) of the gastrointestinal tract (e.g., esophagus). For example, in certain embodiments, the additional active agent also increases the viscosity of the composition, imparts a mucoadhesive character upon the composition and/or enhances absorption of the composition through a surface (e.g., a mucosal or epithelial layer) of the gastrointestinal tract (e.g., esophagus).

In certain embodiments, the acid inhibitor is an H₂RA. H₂RAs includes, by way of non-limiting example, cimetidine, ranitidine, ebrotidine, pabutidine, lafutidine, loxtidine and famotidine. In a specific embodiment, the H₂RA is ranitidine.

In certain embodiments, the acid inhibitor is a PPI. PPIs include, by way of non-limiting example, omeprazole, hydroxyomeprazole, esomeprazole, tenatoprazole, lansoprazole, pantoprazole, rabeprazole, dontoprazole, habeprazole, perprazole, ransoprazole, pariprazole and leminoprazole. In one non-limiting example, the PPI is omeprazole.

In certain embodiments, the corticosteroid is administered in combination with an excipient. In some embodiments, excipients are included in the corticosteroid containing composition to increase the viscosity of the delivered composition. In various embodiments, the liquid viscosity is increased in the oral dosage form of the corticosteroid containing composition. In other embodiments, the excipient increases the viscosity of the oral dosage form of the corticosteroid containing composition once the oral dosage form is dissolved (e.g., in saliva). It is to be understood that in various embodiments of the present invention, the viscosity of the oral dosage form (or of the dissolved oral dosage form) is at a level that is sufficient to deliver an effective amount of the composition to the esophagus. In some embodiments, the effective amount of the composition delivered to the esophagus is an amount sufficient to coat the esophagus, and thereafter deliver the composition to the affected areas, including by way of example only, the lower esophagus, the esophageal-stomach juncture, the stomach, the duodenum and/or within 3 cm of the Z-line. In certain embodiments, the viscosity of the oral dosage form (or of the dissolved oral dosage form) is such that when administered orally, it is not so thick as to cause difficulty in swallowing, cause gagging, or be unpalatable. Those of ordinary skill in the art can determine the viscosity of the compositions provided herein, and may thus determine appropriate ranges. In certain embodiments, the viscosity of the oral dosage form (or of the dissolved oral dosage form) is a viscosity that is sufficient to provide exposure of the corticosteroid to the esophagus for a sufficient period of time such that the symptoms of and/or inflammation associated with GERD are reduced following administration of the corticosteroid containing oral dosage form.

One method for determining sufficient viscosity may include monitoring changes in the interaction of the composition with a surface (e.g., a mucosal or epithelial layer) of the gastrointestinal tract (e.g., esophagus), including but not limited to measuring changes in residence or retention time of the composition in the absence and presence of the excipient. Another method for determining whether the composition is sufficiently viscous is by determining whether the inflammation of the esophagus is reduced after treatment with the composition.

Viscosity can also be determined by any method that will measure the resistance to shear offered by the substance or preparation. Many viscometers are available to those in the pharmaceutical field, and include those built by, for example, Brookfield.

Viscosity-enhancing excipients that may be used in pharmaceutical compositions described herein include, but are not limited to, cellulose or a cellulose derivative, acacia (gum arabic), agar, aluminum magnesium silicate, sodium alginate, sodium stearate, bladderwrack, bentonite, carbomer, carrageenan, Carbopol, cellulose, microcrystalline cellulose (MCC), ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), Splenda® (distributed by McNeil Nutritionals, LLC Fort Washington, PA 19034-2299) or combinations thereof. In one non-limiting example, the viscosity-enhancing excipient is Splenda®. In specific embodiments, the viscosity-enhancing excipient is a combination of MCC and CMC (e.g., Avicel RC-591). In some embodiments, the CMC/MCC combination (e.g., Avicel® RC-591) is present in the composition in an amount of about 1 mg/mL to about 150 mg/mL, 1 mg/mL to about 75 mg/mL, or about 5 mg/mL to about 40 mg/mL. In certain embodiments, the CMC/MCC mixed weight ratio is between about 1/99 and about 99/1, about 20/80 and about 5/95, or about 15/85 and about 10/90. In a specific embodiment, the CMC is NaCMC and the CMC/MCC mixed weight ratio is about 11/89.

In some embodiments, the viscosity of the composition is at least about 1 mPa.s, at least about 2 mPa.s, at least about 3 mPa.s, at least about 5 mPa.s, at least about 10 mPa.s, at least about 15 mPa.s, at least about 20 mPa.s, at least about 25 mPa.s, at least about 30 mPa.s, at least about 35 mPa.s, at least about 40 mPa.s, or at least about 50 mPa.s. In some embodiments, the viscosity of the composition is at least about 100 mPa.s. In certain embodiments, the viscosity of the composition, measured at 25 degrees Celsius, is about 50 mPa.s to about 250,000 mPa.s, about 50 mPa.s to about 70,000 mPa.s, about 50 mPa.s to about 25,000 mPa.s, about 50 mPa.s to about 10,000 mPa.s, about 50 mPa.s to about 3,000 mPa.s or about 50 mPa.s to about 2,000 mPa.s. In one aspect, the viscosity of the composition, as measured at 25 degrees Celsius, is from about 25 mPa.s to about 800 mPa.s, about 50 mPa.s to about 800, or about 300 mPa.s to about 800 mPa.s (e.g., measured by a Brookfield viscometer). In another aspect, the viscosity of the composition may range from about 100 mPa.s to about 200 mPa.s, about 200 mPa.s to about 300 mPa.s, about 250 mPa.s to about 600 mPa.s or about 400 mPa.s to about 600 mPa.s. In specific embodiments, the viscosity of the formulation is about 30 mPa.s, about 100 mPa.s, about 200 mPa.s, about 300 mPa.s, about 400 mPa.s, about 500 mPa.s, or about 250,000 mPa.s (e.g., as measured with a Brookfield viscometer at 25 degrees Celsius equipped with an ultra low adapter). In some embodiments, a composition or formulation described herein comprises a viscosity enhancing agent that imparts on the composition a viscosity sufficient to provide increased residence on the esophagus while also allowing migration of the active agent(s) (solute or particles) when the composition is orally administered to an individual. In other words, in some embodiments, the viscosity is high enough to increase residence time of the composition on a surface (e.g., a mucosal or epithelial layer) of the gastrointestinal tract (e.g., esophagus), but not so high as to prevent migration of the active agent(s) within the composition, e.g., toward the surface (e.g., a mucosal or epithelial layer) of the gastrointestinal tract (e.g., esophagus).

Viscosity can also be determined by any method that will measure the resistance to shear offered by the substance or preparation. Many viscometers are available to those in the pharmaceutical field, and include those built by, for example, Brookfield.

In certain embodiments, a pharmaceutical composition described herein is a non-Newtonian fluid or a Newtonian fluid. In some embodiments, a composition described herein is non-Newtonian. In specific embodiments, the non-Newtonian fluid is a plastic, pseudo-plastic or dilatant non-Newtonian fluid. In some specific embodiments, the non-Newtonian fluid is thixotropic. In certain embodiments, the non-Newtonian fluid composition thins with shear, and thickens upon the absence of shear. Thus, in some embodiments, provided herein is a fluid pharmaceutical composition that is suitable for easy pouring following mild or moderate agitation. Furthermore, in some embodiments, provided herein is a fluid pharmaceutical composition that while being suitable for easy pouring following mild or moderate agitation becomes viscous enough upon oral administration to allow the pharmaceutical composition to at least partially coat the esophagus and topically deliver a therapeutically effective amount of corticosteroid to the esophagus. In some embodiments, the at least one additional excipient is selected from a non-Newtonian viscosity enhancing agent (i.e., an agent that provides a composition herein with a non-Newtonian character). Non-Newtonian viscosity enhancing agents include, by way of non-limiting example, acacia (e.g., used in about 5-10% w/w of a pharmaceutical composition described herein), alginic acid (e.g., about 0.5-20% w/w), carbomer, CaCMC, NaCMC, carrageenan (e.g., about 0.3-12% w/w), ceratonia (e.g., about 0.1-1% w/w), chitosin (e.g., about 0.5-2% w/w), colloidal silicon dioxide (e.g., about 2-10% w/w), ethylcellulose (e.g., about 5-25% w/w), gelatin, guar gum (e.g., about 1-2.5% w/w), HEC, hydroxyethylmethyl cellulose (e.g., about 1-5% w/w), hydroxypropyl cellulose (e.g., about 1-10% w/w), HPMC, magnesium aluminum silicate (e.g., about 2-10% w/w), one or more maltodextrin, methylcellulose (e.g., about 1-2% w/w), polyethylene glycol (e.g., about 45-60% w/w), povidone (e.g., about 10-15% w/w), saponite, sodium alginate (e.g., about 1-5% w/w), sucrose (e.g., about 50-70% w/w), tragacanth (e.g., about 0.1-2% w/w), xanthan gum (e.g., about 0.1-1% w/w), an combinations thereof.

A Newtonian fluid can be described as a fluid whose viscosity is equal to the shear stress exerted by the fluid divided by the velocity gradient perpendicular to the direction of the shear. In certain embodiments, the at least one additional excipient is selected from a Newtonian viscosity enhancing agent (i.e., an agent that provides a composition herein with a Newtonian character). Newtonian viscosity enhancing agents include, by way of non-limiting example, glycerin (e.g., about 50-80% w/w), polydextrose (e.g., about 50-70% w/w), and combinations thereof.

In some embodiments, a pharmaceutical composition described herein is sufficiently spreadable and/or has an appropriate flow characteristic on a surface (e.g., a mucosal or epithelial layer) of the gastrointestinal tract (e.g., esophagus). In certain embodiments, the spreadability and/or flow characteristic of the composition is suitable so as to allow a pharmaceutical composition or a unit dose of a pharmaceutical composition described herein to spread across and/or flow upon a surface (e.g., a mucosal or epithelial layer) of the gastrointestinal tract (e.g., esophagus) and at least partially coat the surface (e.g., a mucosal or epithelial layer) of the gastrointestinal tract (e.g., esophagus). In some embodiments, by at least partially coating the surface (e.g., a mucosal or epithelial layer) of the gastrointestinal tract (e.g., esophagus), topical delivery of the corticosteroid to the gastrointestinal site is achieved.

Excipients, such as, for example, those listed herein, may be included in the composition are mucoadhesive agents including, but not limited to, at least one soluble polyvinylpyrrolidone polymer (PVP); a carbopol; a water-swellable, but water-insoluble, fibrous, cross-linked carboxy-functional polymer; a crosslinked poly(acrylic acid) (e.g. Carbopol 947P); a carbomer homopolymer; a carbomer copolymer; a hydrophilic polysaccharide gum, one or more maltodextrin, a cross-linked alignate gum gel, a water-dispersible polycarboxylated vinyl polymer, at least two particulate components selected from the group consisting of titanium dioxide, silicon dioxide, and clay, or a mixture thereof. The mucoadhesive agent may be used in combination with a viscosity increasing excipient, or may be used alone to increase the interaction of the composition with the esophagus. In certain embodiments, the mucoadhesive agent also imparts an increased viscosity character on the composition (as compared to a composition lacking the mucoadhesive agent). In other embodiments, the mucoadhesive agent does not substantially affect the viscosity of the composition.

In certain embodiments, the mucoadhesive agent and/or viscosity enhancing agent comprises one or more maltodextrin. In various aspects, the physical characteristics of maltodextrins vary depending, e.g., on the dextrose equivalent of the specific maltodextrin. In certain aspects, the dextrose equivalent of a specific maltodextrin may affect the viscosity, hygroscopicity, sweetness, humectancy, plasticity, solubility and or mucoadhesiveness of the maltodextrin. Thus, in various embodiments, a maltodextrin is selected based on the specific character that is desired to be imparted upon the pharmaceutical composition described herein. In certain embodiments, a maltodextrin is selected that increases the mucoadhesive character of a composition described herein without substantially increasing the viscosity of the composition (e.g., compared to an otherwise identical composition lacking the maltodextrin). In other embodiments, a maltodextrin is selected that increases the viscosity of a composition described herein without substantially increasing the mucoadhesiveness of the composition (e.g., compared to an otherwise identical composition lacking the maltodextrin). In some embodiments, the oral pharmaceutical composition comprises a first maltodextrin that increases the viscosity of the oral pharmaceutical composition and a second maltodextrin that increases the mucoadhesive character of the oral pharmaceutical composition (e.g., compared to an otherwise identical composition lacking the second maltodextrin).

In some embodiments, a composition or formulation described herein comprises less than about 0.1 g or less than about 1 g of maltodextrin for every mL of liquid vehicle in the oral pharmaceutical composition. In certain embodiments, a composition or formulation described herein comprises at least one maltodextrin. In certain instances, a composition or formulation described herein comprises less than 2 g of maltodextrin/mL of composition, less than 1.5 g of maltodextrin/mL of composition, less than 1 g of maltodextrin/mL of composition, less than 0.5 g of maltodextrin/mL of composition, less than 0.25 g/mL of maltodextrin/mL of composition, about 0.05 g of maltodextrin/mL of composition to about 0.5 g of maltodextrin/mL of composition, about 0.05 g of maltodextrin/mL of composition to about 0.4 g of maltodextrin/mL of composition, about 0.05 g of maltodextrin/mL of composition to about 0.3 g of maltodextrin/mL of composition, about 0.1 g of maltodextrin/mL of composition to about 0.5 g of maltodextrin/mL of composition, about 0.1 g of maltodextrin/mL of composition to about 0.4 g of maltodextrin/mL of composition, about 0.1 g of maltodextrin/mL of composition to about 0.3 g of maltodextrin/mL of composition, about 0.2 g of maltodextrin/mL of composition to about 0.5 g of maltodextrin/mL of composition, about 0.2 g of maltodextrin/mL of composition to about 0.4 g of maltodextrin/mL of composition, or about 0.2 g of maltodextrin/mL of composition to about 0.3 g of maltodextrin/mL of composition. In some embodiments, any composition or formulation described herein comprises greater than about 7% w/w, greater than about 8% w/w, greater than about 9% w/w, greater than about 10% w/w, greater than about 11% w/w, greater than about 12% w/w, greater than about 13% w/w, greater than about 14% w/w, greater than about 15% w/w, greater than about 16% w/w, greater than about 17% w/w, greater than about 18% w/w, greater than about 19% w/w, greater than about 20% w/w, greater than about 21% w/w, greater than about 22% w/w, greater than about 23% w/w, greater than about 24% w/w, greater than about 25% w/w, greater than about 26% w/w, greater than about 27% w/w, greater than about 28% w/w, greater than about 29% w/w or greater than about 30% w/w of maltodextrin. In specific embodiments, the maltodextrin is substantially dissolved in a liquid vehicle of the composition or formulation. In certain embodiments, the maltodextrin has a dextrose equivalents (DE) of greater than 4, greater than 5, greater than 10, greater than 11, greater than 12, greater than 13, greater than 14, greater than 15, about 15, about 4 to about 10, about 4 to about 9, about 4 to about 8, about 11 to about 20, about 12 to about 19, about 13 to about 18, or about 14 to about 16. In specific embodiments, the first maltodextrin has a DE of about 4 to about 10, about 4 to about 9, or about 4 to about 8 and the second maltodextrin has a DE of about 10 to about 20, about 12 to about 19, or about 13 to about 18. In some embodiments, at least one maltodextrin utilized in a composition described herein has a molecular weight high enough to increase the solubility of a corticosteroid, or to increase the suspendability of a corticosteroid particle.

In some embodiments, an excipient that enhances the interaction of a composition or formulation described herein (e.g., maltodextrin) is substantially dissolved in a liquid vehicle of the composition or formulation.

In certain instances, the mucoadhesive character and/or viscosity imparted to a composition described herein is sufficient to deliver an effective amount of the composition to, for example, the esophagus in an amount that may coat the esophagus, and thereafter deliver the composition to the affected areas, including by way of example only, the lower esophagus, the esophageal-stomach juncture, the stomach, the duodenum and/or within 3 cm of the Z-line. Also, in some instances, the mucoadhesive character and/or viscosity is at a level that may be given orally, i.e. allows a patient to swallow, limits a gagging reaction, and is palatable. Those of ordinary skill in the art can determine the mucoadhesive characteristics of the compositions provided herein, and may thus determine appropriate ranges. One method for determining sufficient mucoadhesiveness may include monitoring changes in the interaction of the composition with a surface (e.g., a mucosal or epithelial layer) of the gastrointestinal tract (e.g., esophagus), including but not limited to measuring changes in residence or retention time of the composition in the absence and presence of the excipient. Another method for determining whether the composition is sufficiently mucoadhesive is by determining whether the inflammation of the esophagus is reduced after treatment with the corticosteroid.

Mucoadhesive agents have also been described, for example, in U.S. Patent Nos. 6,638,521, 6,562,363, 6,509,028, 6,348,502, 6,319,513, 6,306,789, 5,814,330, and 4,900,552.

In another non-limiting example, a mucoadhesive agent can be, for example, at least two particulate components selected from titanium dioxide, silicon dioxide, and clay, wherein the composition is not further diluted with any liquid prior to administration and the level of silicon dioxide, if present, is from about 3% to about 15%, by weight of the composition. Silicon dioxide, if present, may be selected from the group consisting of fumed silicon dioxide, precipitated silicon dioxide, coacervated silicon dioxide, gel silicon dioxide, and mixtures thereof. Clay, if present, may be kaolin minerals, serpentine minerals, smectites, illite or a mixture thereof. For example, clay can be laponite, bentonite, hectorite, saponite, montmorillonites or a mixture thereof.

Excipients, such as, for example, those listed herein, that may be included in the composition are absorption enhancing agents. Examples of absorption enhancing include, but are not limited to, acylcarnitines, surfactants, sodium lauryl sulfate, saponins, bile salts or bile acids including but not limited to cholanic acid, chilic acid, deoxycholic acid, glycocholic acid, tautocholic acid, chenodeoxycholic acid, lithocholic acid, ursocholic acid, ursodeoxycholic acid, isourosde oxycholic acid, lagodeoxycholic acid, glycodeoxycholic acid, glycochenodeoxycholic acid, dehydrocholic acid, hyocholic acid, hyodeoxycholic acid, or combinations thereof, dihydrofusidates, fatty acid derivatives, chitosan, carbopol, cellulosic agents, sterols, including but not limited to alcohols structurally related to steroids, including but not limited to cholestanol, coprostanol, cholesterol, epicholesterol, ergosterol, ergocalciferol, or combinations thereof, starch, dextran, cyclodextrin, or combinations thereof. Absorption enhancing agents may act by increasing absorption of the active agent, including corticosteroids and acid inhibitors, through a surface (e.g., a mucosal or epithelial layer) of the gastrointestinal tract (e.g., esophagus). Examples of absorption enhancing agents are disclosed in WO 2005/113008.

The compositions contemplated herein may also include a combination of excipients that are viscosity enhancing agents, mucoadhesive agents and/or absorption enhancing agents. Moreover, an excipient may exhibit multiple characteristics, i.e. may be both a viscosity enhancing agent and a mucoadhesive agent. The composition may also include excipients that do not impart characteristics of viscosity enhancing, mucoadhesive agents or absorption enhancing activity.

In certain embodiments, a composition provided herein comprises or is prepared by combining the components set forth in any of Tables 1-13. In various embodiments, one or more of maltodextrin, dextrose, HEC, CMC, MCC, Carbomer and HPMC are utilized therein.

**Table 1: Budesonide Composition #1**

| **Ingredient** | **Amount** |
|---|---|
| Budesonide | 1 mg to 150 mg |
| CMC, MCC, Carbomer, HPMC and/or HEC | 0.5 g to 10 g |
| Dextrose | 0 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g, Tween 80) | 5 mg to 100 mg |
| Flavoring Agent | optional |
| Sweetener | optional |
| Preservative | optional |
| Water | q.s. to 100 mL |

**Table 2: Budesonide Composition #2**

| **Ingredient** | **Amount** |
|---|---|
| Budesonide | 1 mg to 150 mg |
| CMC, MCC, Carbomer, HPMC and/or HEC | 0 g to 10 g |
| Dextrose | 1 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 3: Budesonide Composition #3**

| **Ingredient** | **Amount** |
|---|---|
| Budesonide | 1 mg to 150 mg |
| CMC, MCC, Carbomer, HPMC and/or HEC | 0 g to 10 g |
| Dextrose | 0 g to 100 g |
| Maltodextrin | 1 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 4: Budesonide Composition #4**

| **Ingredient** | **Amount** |
|---|---|
| Budesonide | 0.5 mg to 2 mg |
| CMC and MCC (e.g., Avicel RC-591) | 0.01 g to 0.3 g |
| Dextrose | 0.1 g to 1 g |
| Maltodextrin | 0.5 g to 2 g |
| EDTA (e.g., disodium edetate) | 1 mg to 10 mg |
| Citric Acid | 0.1 mg to 100 mg |
| Citrate (e.g., sodium citrate) | 0.1 mg to 200 mg |
| Polysorbate 80 (e.g., Tween 80) | 0.1 mg to 10 mg |
| Cherry Flavor | 1 mg to 100 mg |
| Sweetener | 100 mg to 1 g |
| Sodium Benzoate | 1 mg to 50 mg |
| Potassium Sorbate | 1 mg to 50 mg |
| Water | q.s. to 5 mL |

**Table 5: Budesonide Composition #5**

| **Ingredient** | **Amount** |
|---|---|
| Budesonide | 0.5 mg to 2 mg |
| CMC and MCC (e.g., Avicel RC-591) | 0.02 g to 0.6 g |
| Dextrose | 0.2 g to 2 g |
| Maltodextrin | 1 g to 4 g |
| EDTA (e.g., disodium edetate) | 2 mg to 20 mg |
| Citric Acid | 0.2 mg to 200 mg |
| Citrate (e.g., sodium citrate) | 0.2 mg to 400 mg |
| Polysorbate 80 (e.g., Tween 80) | 0.2 mg to 20 mg |
| Cherry Flavor | 2 mg to 200 mg |
| Sweetener | 200 mg to 2 g |
| Sodium Benzoate | 2 mg to 100 mg |
| Potassium Sorbate | 2 mg to 100 mg |
| Water | q.s. to 10 mL |

**Table 6: Budesonide Composition #6**

| **Ingredient** | **Amount** (mg/mL) |
|---|---|
| Budesonide | 0.01 to 0.5 |
| CMC and MCC (e.g., Avicel RC-591) | 2 to 100 |
| Dextrose | 10 to 500 |
| Maltodextrin (M150) | 10 to 500 |
| EDTA (e.g., disodium edetate) | 0.01 to 10 |
| Citric acid | 0.1 to 10 |
| Citrate (e.g., sodium citrate) | 0.1 to 10 |
| Polysorbate 80 (e.g., Tween 80) | 0.01 to 1 |
| Flavoring agent (e.g., Cherry Flavor) | 0.1 to 100 |
| Glycerin | 10 to 100 |
| Acesulfame potassium | 0.1 to 40 |
| Magnasweet 110 | 0.1 to 40 |
| Sodium Benzoate | 0.1 to 10 |
| Potassium Sorbate | 0.1 to 10 |
| Water | q.s. to 1-15 mL |

**Table 7: Budesonide Composition #7**

| **Ingredient** | **Amount** (mg/mL) |
|---|---|
| Budesonide | about 0.05 to about 0.2 |
| CMC and MCC (e.g., Avicel RC-591) | 5 to 50 |
| Dextrose | 50 to 250 |
| Maltodextrin (M150) | 200 to 500 |
| EDTA (e.g., disodium edetate) | 0.1 to 1 |
| Citric acid | 0.5 to 5 |
| Citrate (e.g., sodium citrate) | 0.2 to 2 |
| Polysorbate 80 (e.g., Tween 80) | 0.01 to 0.4 |
| Flavoring agent (e.g., Cherry Flavor) | 1 to 10 |
| Glycerin | 30 to 80 |
| Acesulfame potassium | 1 to 10 |
| Magnasweet 110 | 1 to 10 |
| Sodium Benzoate | 0.5 to 4 |
| Potassium Sorbate | 0.5 to 4 |
| Water | q.s. to 1-15 mL |

**Table 8: Budesonide Composition #8**

| **Ingredient** | **Amount** (mg/mL) | **Amount** % w/w |
|---|---|---|
| Budesonide | 0.05 | 0.004 |
| Avicel RC-591 | 23.6 | 2 |
| Dextrose | 118 | 10 |
| Maltodextrin (M150) | 306.8 | 26 |
| Disodium edetate | 0.59 | 0.05 |
| Citric acid | 1.77 | 0.15 |
| Sodium citrate | 0.59 | 0.05 |
| Polysorbate 80 | 0.12 | 0.01 |
| Cherry Flavor | 5.9 | 0.5 |
| Glycerin | 59 | 5 |
| Acesulfame potassium | 5.9 | 0.5 |
| Magnasweet 110 | 5.9 | 0.5 |
| Sodium Benzoate | 2.36 | 0.2 |
| Potassium Sorbate | 2.36 | 0.2 |
| Water | q.s. to 1, 2, 3, 4, 5, 6, 7, 8, 9, 10, 11, 12, 13, 14,or 15 mL | q.s. to 1, 2, 3, 4, 5, 6, 7, 8, 9, 11, 12, 13, 14,or 15 mL |

**Table 9: Budesonide Composition #9**

| **Ingredient** | **Amount** (mg/mL) | **Amount** % w/w |
|---|---|---|
| Budesonide | 0.2 | 0.17 |
| Avicel RC-591 | 23.6 | 2 |
| Dextrose | 118 | 10 |
| Maltodextrin (M150) | 306.8 | 26 |
| Disodium edetate | 0.59 | 0.05 |
| Citric acid | 1.77 | 0.15 |
| Sodium citrate | 0.59 | 0.05 |
| Polysorbate 80 | 0.12 | 0.01 |
| Cherry Flavor | 5.9 | 0.5 |
| Glycerin | 59 | 5 |
| Acesulfame potassium | 5.9 | 0.5 |
| Magnasweet 110 | 5.9 | 0.5 |
| Sodium Benzoate | 2.36 | 0.2 |
| Potassium Sorbate | 2.36 | 0.2 |
| Water | q.s. to 1, 2, 3, 4, 5, 6, 7, 8, 9, 10, 11, 12, 13, 14,or 15 mL | q.s. to 1, 2, 3, 4, 5, 6, 7, 8, 9, 10, 11, 12, 13, 14,or 15 mL |

**Table 10: Fluticasone Propionate Composition #1**

| **Ingredient** | **Amount** |
|---|---|
| Fluticasone Propionate | 0.5 mg to 150 mg |
| CMC, MCC, Carbomer, HPMC and/or HEC | 0.5 g to 10 g |
| Dextrose | 0 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 11: Fluticasone Propionate Composition #2**

| **Ingredient** | **Amount** |
|---|---|
| Fluticasone Propionate | 0.5 mg to 150 mg |
| CMC, MCC, Carbomer, HPMC and/or HEC | 0 g to 10 g |
| Dextrose | 1 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 12: Fluticasone Propionate Composition #3**

| **Ingredient** | **Amount** |
|---|---|
| Fluticasone Propionate | 0.5 mg to 150 mg |
| CMC, MCC, Carbomer, HPMC and/or HEC | 0 g to 10 g |
| Dextrose | 0 g to 100 g |
| Maltodextrin | 1 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 13: Corticosteroid Composition**

| **Ingredient** | **Amount % w/w** |
|---|---|
| Corticosteroid | 0.001 to 1 |
| Sodium methylparaben | 0.0001 to 0.1 |
| Sorbitol | 5 to 30 |
| Sucrose | 1 to 40 |
| Corn starch | 1 to 10 |
| MCC | 0.1 to 5 |
| CMC (NaCMC) | 0.1 to 5 |
| Xanthan | 0.001 to 1 |
| Glycerin | 0.1 to 10 |
| Calcium carbonate | 0 to 30 |
| Magnesium hydroxide | 0 to 5 |
| Color (e.g., FD&C Red No. 3) | optional |
| Water | q.s. to 1, 2, 3, 4, 5, 6, 7, 8, 9, 10, 11, 12, 13, 14,or 15 mL |

In certain embodiments, compositions provided herein are used in methods of treating GERD. In some embodiments, the present invention provides for pharmaceutical compositions comprising a therapeutically effective amount of a corticosteroid (e.g., a topical corticosteroid, such as budesonide or fluticasone). In some embodiments, the pharmaceutical compositions used herein comprise a therapeutically effective amount of a corticosteroid and a therapeutically effective amount of an acid inhibitor. In specific embodiments, the acid inhibitor is selected from, e.g., a PPI, a H₂RA or a combination thereof. In some embodiments, the pharmaceutical compositions described herein further comprise an excipient, or combination of excipients, that increases the interaction of the composition with the esophagus or target area. In specific embodiments, the excipient or excipients impart an increased viscosity on the composition, an increased mucoadhesive character on the composition, or a combination thereof.

In various embodiments of the present invention, the pharmaceutical compositions provided herein are in liquid form. Liquid forms include, by way of non-limiting example, neat liquids, solutions, suspensions, dispersions, colloids and the like. In other embodiments, the pharmaceutical compositions provided herein are in the form of a dissolvable oral dosage form. Dissolvable oral dosage forms include any pharmaceutically acceptable oral dosage form that becomes fluid upon contact with saliva. Dissolvable oral dosage forms include, by way of non-limiting example, lozenges, tablets, dissolving wafers, capsules, or gel capsules. In some embodiments, a pharmaceutical composition described herein is in liquid, semi-solid or solid form. In specific embodiments, a pharmaceutical composition described herein is in semi-solid form, e.g., a gel, a gel matrix, a cream, a paste, or the like. In some embodiments, semi-solid forms comprise a liquid vehicle.

In some embodiments, any composition or formulation described herein is stable. In specific embodiments, the composition is chemically and physically stable. In certain embodiments, chemical stability is evidenced by a composition that comprises at least 80%, 90%, 95%, 98%, or 99% of the initial amount or label amount of corticosteroid and/or optional additional active agent therein for, by way of non-limiting example, 1 week, 2 weeks, 3 weeks, 1 month, 3 months, 6 months, 1 year, 2 years, or for the duration of the shelf life. In some embodiments, physical stability is evidenced by a pharmaceutical composition that is able to substantially obtain uniformity, remain substantially uniform (e.g., for at least 1 day, 3 days, 1 week, 2 weeks, 3 weeks, 1 month, 3 months, 6 months, 1 year, 2 years, etc.), or substantially regain uniformity (e.g., via mild or moderate agitation after being undisturbed for 1 day, 3 days, 1 week, 2 weeks, 3 weeks, 1 month, 3 months, 6 months, 1 year, 2 years, etc.). In certain embodiments, physical stability is evidenced by a composition that comprises at least 80%, 90%, 95%, 98%, or 99% of the initial amount or label amount of corticosteroid and/or optional additional active agent therein for, by way of non-limiting example, 2 days, 1 week, 2 weeks, 3 weeks, 1 month, 3 months, 6 months, 1 year, 2 years, or for the duration of the shelf life. In certain embodiments, uniformity as described herein is evidenced by the uniformity of the dispersion of the corticosteroid particles throughout the pharmaceutical composition, the uniformity of the dispersed mass of corticosteroid throughout the pharmaceutical composition, the uniformity of the concentration of one or more of the components in the composition throughout the pharmaceutical composition, and the like. In certain embodiments, mild or moderate agitation includes, by way of non-limiting example, shaking, shaking well, swirling, gentle swirling, and the like. In some embodiments, mild or moderate agitation includes agitation without a special apparatus. In some embodiments, uniformity of the pharmaceutical composition refers to dose uniformity (e.g., each dose delivered or withdrawn from the composition comprises a substantially similar amount of corticosteroid), or the concentration of corticosteroid in at least some or all of the doses from the multiple dose formulations are substantially similar. In certain embodiments, substantially similar includes, e.g., within 20%, 15%, 10%, 7%, 5%, 3%, 2%, or 1%.

In various embodiments, these pharmaceutical compositions are used in treating GERD in an individual by administering such compositions to an individual. In certain embodiments, the individual is an individual in need of treatment (i.e. an individual suffering from GERD). As used herein, the term "individual" includes any animal. In some embodiments, the animal is a mammal. In certain embodiments, the mammal is a human. In specific embodiments, the human is an adult. In other embodiments, the human is a child or infant. In certain embodiments, the child or infant is less than 16 years old, less than 12 years old, less than 8 years old, less than 6 years old, less than 4 years old or less than 2 years old. In certain embodiments, the individual is an individual suffering from symptoms of or inflammation associated with GERD. In some embodiments, the individual is a patient in need of a therapy for the treatment of symptoms of or inflammation associated with GERD.

### Formulations

In certain embodiments, the methods provided herein are used in therapies for the treatment of animals. In some embodiments, the methods provided herein are used in the treatment of humans, primates or domesticated animals.

Pharmaceutical compositions suitable for use in the present invention include compositions wherein the active ingredient(s) is/are contained in an effective amount to achieve the intended purpose. In certain embodiments, the therapeutically effective amount of an active used in either a pharmaceutical composition or a method described herein is an amount sufficient to provide a therapeutically beneficial effect. In specific embodiments, the therapeutically beneficial effect is the prevention of, the reduction in the incidences of, the alleviation of, the relief from or the treatment of a symptom of or inflammation associated with GERD. In light of the discussion provided herein, determination of the effective amounts is well within the capability of those skilled in the art.

The exact dosage will depend upon the route of administration, the form in which the composition is administered, the subject to be treated, the age, body weight/height of the subject to be treated, and the preference and experience of the attending physician. In certain embodiments, the optimal concentration of the corticosteroid in the composition depends upon the specific corticosteroid used, the characteristics of the patient, and the nature of the inflammation for which the treatment is sought. In various embodiments, these factors are determined by those of skill in the medical and pharmaceutical arts in view of the present disclosure.

Generally, a therapeutically effective dose of the active(s) is desired. A therapeutically effective dose refers to the amount of the active(s) that results in a degree of amelioration of symptoms and/or inflammation relative to the status of such symptoms and/or inflammation prior to treatment. The dosage forms and methods of applying dosage forms containing effective amounts are within the scope of the instant invention.

In various embodiments, the amount of corticosteroid (e.g., budesonide or fluticasone propionate) used in a method or in a composition described herein is from about 2.5 to 400 µg/kg of body weight per day, or for example, in the range of 5 to 300 µg/kg per day, or for example in the range of 5 to 200 µg/kg per day, or for example in the range of 5 to 100 µg/kg per day, or for example in the range of 10 to 100 µg/kg per day, or for example in the range of 10-50 µg/kg per day, or for example in the range of 10-100 µg/kg/day, or for example in the range of 5-50 µg/kg/day, or in an illustrative embodiment in the range of 10-60 µg/kg/day. In some embodiments, the amount of corticosteroid (e.g., budesonide or fluticasone propionate) used in a method, in a combination or a dose of a combination disclosed herein includes, by way of non-limiting example, about 100 ug to about 20 mg, about 300 ug to about 4 mg, about 50 µg to about 500 mg, about 50 µg to about 200 mg, about 50 µg to about 100 mg, about 50 µg to about 50 mg, about 250 µg to about 20 mg, about 250 µg to about 15 mg, about 250 µg to about 10 mg, about 250 µg to about 5 mg, about 250 µg to about 3 mg, or about 500 µg to about 3 mg, about 375 µg to about 1.5 mg, or about 500 µg to about 2 mg, or about 1 mg to about 3 mg. In an illustrative embodiment, the dosage is provided in a sufficient volume to allow the composition to reach the esophagus in an effective amount. In some embodiments, a composition described herein comprises 1 or more doses. In certain embodiments, a composition described herein is contained in a multiple unit container. Thus, provided herein is a kit comprising a composition described herein and a container (e.g., a multiple unit or single unit container). In certain embodiments, provided herein is a composition or a kit comprising a composition that comprises from about 2 and about 180, about 10 to about 60, about 14 or about 30 doses.

In certain embodiments of the present invention, the corticosteroid is provided in the form of a lozenge. In some embodiments, the lozenge is dissolved in the mouth, thus reaching and coating the esophagus. In various embodiments, the lozenge or other similar tablet, capsule, or other solid, dissolve rapidly in the mouth or esophagus to produce a dissolved oral dosage form (e.g., a solution) that can then coat the esophagus. In other embodiments, e.g., for children or other patients that may have difficulty with a dissolving lozenge, the lozenge is ground or otherwise dissolved in a small volume of water or other pharmaceutically suitable liquid, which is then administered. In other illustrative embodiments of the invention, the corticosteroid is provided in the form of a tablet, a capsule, or, for example a gel capsule, designed for slow release and delivery to the esophagus.

In some embodiments, initial treatment continues, for example, for about 3 days to 2 weeks for an acute condition, or about 4 weeks to about 16 weeks for a chronic condition, or about 8 weeks to about 12 weeks for a chronic condition. In various embodiments, longer therapy is needed, such as, for example, therapy similar to chronic therapy for persistent asthma. In some aspects of the present invention, patients are, for example, be treated for up to 6 months, or up to one year. In certain aspects, maintenance treatment last up to or longer than one year. In some embodiments, patients are treated on a maintenance basis or on an as needed basis during a problematic episode, depending on the severity of the condition. In certain embodiments, patients are treated on a rotating treatment basis, where treatment is provided for a period of time and then the patient is taken off of the drug for a period before treatment resumes again. When off the drug, the patient may be given no treatment, treatment with another medication, or treatment with a reduced dosage. In certain embodiments, patients are given treatment with a higher dose of the composition until a desired reduced disease state is achieved, and then continued on a lower dose of the composition.

In one embodiment of the instant invention, specific excipients that may effect viscosity and increase interaction with a surface (e.g., a mucosal or epithelial layer) of the gastrointestinal tract (e.g., esophagus), may be included in the composition. Such viscosity enhancing agents include but are not limited to, acacia (gum arabic), agar, aluminum magnesium silicate, sodium alginate, sodium stearate, bladderwrack, bentonite, carbomer, carrageenan, Carbopol, cellulose, microcrystalline cellulose, ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500) gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose, sodium carboxymethyl-cellulose (CMC), silicon dioxide, polyvinylpyrrolidone (PVP: povidone), Splenda® (dextrose, maltodextrin and sucralose) or combinations thereof. In certain embodiments, a viscosity-increasing excipient that may be used is Splenda®. In specific embodiments, the viscosity-enhancing excipient is a combination of MCC and CMC (e.g., Avicel RC-591).

In one aspect, viscosity of the composition is from about 2 mPa.s or greater, or about 25 mPa.s to about 800 mPa.s, as measured with a Brookfield viscometer at 25 degrees Celsius, more preferably at about 50 mPa.s to about 800, or about 300 mPa.s to about 800 mPa.s. In another aspect, a viscosity of the composition may range from about 250 mPa.s, to about 600 mPa.s or about 400 mPa.s to about 600 mPa.s. In specific embodiments, the viscosity of the formulation is about 40 mPa.s, about 35 mPa.s, or about 400 mPa.s or about 500 mPa.s, as measured with a Brookfield viscometer at 25 degrees Celsius. A non-limiting example of a viscosity measurement within the parameters disclosed herein is exemplified in a suspension prepared by adding about 5 to about 15 grams of Splenda® to 4 ml of water, or a suspension prepared by adding about 10 to about 12 grams of Splenda to 4 ml of water, wherein the viscosity is measured with a Brookfield viscometer at 25 degrees Celsius. In certain embodiments, viscosities are measured at a shear of about 13.2 sec⁻¹.

In another aspect, excipients that impart mucoadhesive characteristics to a composition, thereby increasing interaction of the composition with a surface (e.g., a mucosal or epithelial layer) of the gastrointestinal tract (e.g., esophagus), are also included. Specific mucoadhesive agents may be used as an excipient, including, but are not limited to, at least one soluble polyvinylpyrrolidone polymer (PVP); a water-swellable, but water-insoluble, fibrous, cross-linked carboxy-functional polymer, a crosslinked poly(acrylic acid) (e.g. Carbopol 947P), a carbomer homopolymer, a carbomer copolymer, a hydrophilic polysaccharide gum, maltodextrin, a cross-linked alignate gum gel, a water-dispersible polycarboxylated vinyl polymer, at least two particulate components selected from the group consisting of titanium dioxide, silicon dioxide, and clay, or a mixture thereof.

In yet another aspect, agents that enhance absorption of the composition through a surface (e.g., a mucosal or epithelial layer) of the gastrointestinal tract (e.g., esophagus), may be used to increase the interaction of the compositions disclosed herein with a surface (e.g., a mucosal or epithelial layer) of the gastrointestinal tract (e.g., esophagus). Such agents include, but are not limited to, acylcarnitines, surfactants, sodium lauryl sulfate, saponins, bile salts or bile acids including but not limited to cholanic acid, chilic acid, deoxycholic acid, glycocholic acid, tautocholic acid, chenodeoxycholic acid, lithocholic acid, ursocholic acid, ursodeoxycholic acid, isourosde oxycholic acid, lagodeoxycholic acid, glycodeoxycholic acid, glycochenodeoxycholic acid, dehydrocholic acid, hyocholic acid, hyodeoxycholic acid, or combinations thereof, dihydrofusidates, fatty acid derivatives, chitosan, carbopol, cellulosic agents, sterols, including but not limited to alcohols structurally related to steroids, including but not limited to cholestanol, coprostanol, cholesterol, epicholesterol, ergosterol, ergocalciferol, or combinations thereof, starch, dextran, cyclodextrin, or combinations thereof.

In other embodiments, the excipient used is a mucoadhesive agent, in others a viscosity enhancing agent, and in yet other embodiments the excipient used as an absorption enhancer. It is also contemplated that the excipient used is a combination of one or more of these agents, or alternatively may not include a mucoadhesive agent, viscosity enhancing or an absorption enhancing agent as the excipient.

In certain embodiments, following administration of a composition or formulation described herein to a surface (e.g., a mucosal or epithelial layer) of the gastrointestinal tract (e.g., esophagus), at least 1%, 2%, 3%, 4%, 5%, 6%, 7%, 8%, 9%, 10%, 15%, 20%, 30%, 40%, 50%, 60%, 70%, 80%, 90% or 95% by weight of the corticosteroid administered adheres to, resides on and/or is absorbed at a surface (e.g., a mucosal or epithelial layer) of the gastrointestinal tract (e.g., esophagus) after at least 5 seconds, 10 seconds, 0.25, 0.5, 0.75, 1, 1.5, 2, 3,4, 5, 6, 7, 8, 9, 10, 15, 20,25, 30, 35, 40, 45, 50, 55 or 60 minutes following application of the composition to the surface (e.g., a mucosal or epithelial layer) of the gastrointestinal tract (e.g., esophagus). In specific embodiments, the surface (e.g., a mucosal or epithelial layer) of the gastrointestinal tract (e.g., esophagus) is the site of gastrointestinal inflammation. In some embodiments, one or more excipient that increases the interaction of the composition with a surface (e.g., a mucosal or epithelial layer) of the gastrointestinal tract (e.g., esophagus) is selected and selected in an amount sufficient to cause a composition or formulation described herein to cause at least 1%, at least 2%, at least 3%, at least 4%, at least 5%, at least 6%, at least 7%, at least 8%, at least 9%, at least 10%, at least 15%, at least 20%, at least 30%, at least 40%, at least 50%, at least 60%, at least 70%, at least 80%, at least 90% or at least 95% by weight of the corticosteroid containing composition or formulation or the corticosteroid to adhere to or reside on a surface (e.g., a mucosal or epithelial layer) of the gastrointestinal tract (e.g., esophagus) for or 5 seconds, 10 seconds, or 0.25, 0.5, 0.75, 1, 1.5, 2, 3, 4, 5, 6, 7, 8, 9, 10, 15, 20, 25, 30, 35, 40, 45, 50, 55 or 60 minutes is after administration to the surface (e.g., a mucosal or epithelial layer) of the gastrointestinal tract (e.g., esophagus).

In specific embodiments, following oral administration of a composition described herein to the esophagus (e.g., following initial swallowing or drinking of the composition), at least 1%, 2%, 3%, 4%, 5%, 6%, 7%, 8%, 9%, 10%, 15%, 20%, 30%, 40%, 50%, 60%, 70%, 80%, 90% or 95% by weight of the corticosteroid or composition administered is present within the esophagus (e.g., as measured by gamma scintigraphy) after at least 5 seconds, 10 seconds, 15 seconds, 20 seconds, 25 seconds, 30 seconds, 40 seconds, 45 seconds, 50 seconds, or 1, 1.5, 2, 3, 4, 5, 6, 7, 8, 9, 10, 15, 20, 25, 30, 35, 40, 45, 50, 55 or 60 minutes following application of the composition to the esophagus. In certain instances, even small differences (e.g., increases) in adherence times (e.g., residence times) between formulations can result in therapeutically significant or clinically significant results or improvements.

One embodiment of the invention includes the use of liquid suspensions of the compositions disclosed herein. In some embodiments, liquid suspensions include a unit dosage form of a composition comprising a corticosteroid. In certain embodiments, liquid suspensions include a unit dosage form of a combination of a corticosteroid and an optional additional active agent (e.g., acid inhibitor). In some embodiments, liquid suspensions include a unit dosage form of a combination of a corticosteroid, additional active agent (e.g., acid inhibitor) and an excipient that increases the interaction of the compositions disclosed herein with a surface (e.g., a mucosal or epithelial layer) of the gastrointestinal tract (e.g., esophagus). Unit dosage forms include the bulk preparation of a composition disclosed herein, such as multiple doses of a liquid suspension contained in a single container or vial. Unit dosage forms may also include doses of a corticosteroid, the combination of a corticosteroid and an additional active agent (e.g., acid inhibitor), or the combination of a corticosteroid, an additional active agent (e.g., acid inhibitor) and an excipient disclosed herein in individual vials or containers. Alternatively, liquid suspensions may include multiple unit dosage forms of a corticosteroid, the combination of a corticosteroid and an additional active agent (e.g., acid inhibitor), or the combination of a corticosteroid, an additional active agent (e.g., acid inhibitor) and an excipient disclosed herein. Multiple unit dosage forms may include the liquid formulations of the individual active agents, e.g. a corticosteroid, or a corticosteroid and an additional active agent (e.g., acid inhibitor). For example, liquid suspensions of the present invention may include those prepared by adding about 5 to about 25 grams of Splenda®, or about 7 to about 20 grams of Splenda®, or about 5 to about 15 grams of Splenda®, or about or about 7 to about 15 grams of Splenda®, or about 8 to about 12 grams of Splenda®, or about 10 to about 11 grams of Splenda®, or 1, 2, 3, 4, 5, 6, 7, 8, 9, 10, 11, 12, 13, 14, 15, 16, 17, 18, 19, 20, 21, 22, 23, 24, or 25 grams of Splenda®, added to 4 ml of budesonide, such as that obtained from a Budesonide respule, or larger volumes having the same ratios of Splenda® to budesonide. Alternatively the liquid suspension may include the formulation above and an additional active agent (e.g., acid inhibitor such as omeprazole).

In other embodiments of the inventions, the formulation may include tablets or capsules for administration to a patient. A tablet or capsule may contain anywhere from 1 mg to as much as 1 g of the active agents, including a corticosteroid, or a corticosteroid and an acid inhibitor. Compositions can be provided in a unit dose formulation for oral administration of a patient. In other embodiments, the table or capsule may be in the form of multiple unit form dosages. In certain embodiments, oral dosage forms of the present invention include between 1 mg and 1 g of an acid inhibitor. In some embodiments, the oral dosage form is a single unit dosage form. In other embodiments, the oral dosage form is a metered dosage form, wherein each metered dose (i.e. the unit dose) includes between 1 mg and 1 g of an acid inhibitor. Likewise, certain embodiments of the present invention provide for methods comprising the administration of between 1 mg and 1 g of an acid inhibitor.

In one aspect, an H₂RA is present in the unit dose in an amount of between 1 mg and 500 mg in combination with a corticosteroid at about 500 µg to 3 mg. In another aspect, a PPI is present in the unit dose with a corticosteroid in an amount of between 1 mg and 600 mg. In yet another aspect, a H₂RA and/or PPI is present in an amount effective to alleviate gastrointestinal reflux by about 2-fold, 3-fold, 4-fold, 5-fold, 10-fold, 20-fold, 30-fold, 50-fold, 100-fold or more (or any fold therebetween).

In specific embodiments, the amount of H₂RA included in either the oral dosage form or in the method described herein is selected from, by way of non-limiting example: cimetidine, 100 to 800 mg/unit dose; ranitidine, 50-300 mg/unit dose; famotidine, 5-100 mg/unit dose; ebrotidine 400-800 mg/unit dose; pabutidine 40 mg/unit dose; lafutidine 5-20 mg/unit dose; and nizatidine, 50-600 mg/unit dose.

In certain embodiments, the amount of PPI included in either the oral dosage form or in the method described herein is from about 5 mg to 600 mg per unit dose. In specific embodiments, the PPI omeprazole is present in an amount from 5 to 50 mg, with about 20 mg per unit dosage form being preferred. In other embodiments, the amount of PPI included in either a oral dosage form or in a method described herein is, by way of non-limiting embodiment: esomeprazole, 5-100 mg/unit dose; lansoprazole, 15-150 mg/unit dose; pantoprazole, 10-200 mg/unit dose; and rabeprazole, 5-100 mg/unit dose. In more specific embodiments, esomeprazole is included in an amount of about 40 mg/unit dose. In another specific embodiment, lansoprazole is included in an amount of about 30 mg/unit dose. In still another specific embodiment, pantoprazole is included in an amount of about 50 mg/unit dose. In yet another specific embodiment, rabeprazole is included in an amount of about 40 mg/unit dose.

The formulation may also be coated with an enteric coating, which protects an active agent, for example a PPI, from degradation in an acidic environment, such as the stomach, and allows a delayed release into a target area, for example the duodenum, for uptake. The enteric coating may be, for example, methacrylate copolymer (for example, Eudragit L100 and Eudragit L100-55), hydroxypropylmethyl cellulose phthalate, hydroxypropylmethyl cellulose acetate succinate, carboxymethylethyl cellulose, acetate phthalate (CAP), cellulose acetate trimellitate (CAT), hydroxypropylmethylcellulose phthalate (HPMCP), copolymer of methacrylic acid and ethyl acrylate, hydroxypropylmethylcellulose acetate succinate (HPMCAS), shellac, chitosan succinate, chitosan phthlate, cellulose acetate trimelliate and polyvinyl acetate phthalate (PVAP), or combinations thereof. A sustained-release substrate may also be used, such as methacrylic acid polymers [e.g., Eudragit NE30D (trade name), Eudragit RL30D (trade name), Eudragit RS30D (trade name), etc.]; water-soluble polymers; plasticizers such as triethyl citrate, polyethylene glycol, acetylated monoglycerides, triacetine alkyl celluloses, e.g. carboxymethylcellulose, other cellulosic materials or compounds (e.g., cellulose acetate phthalate, hydroxypropylmethylcellulose phthalate), methyl cellulose, ethyl cellulose or propyl cellulose, more preferably ethyl cellulose. polyvinyl acetate polymers (e.g., polyvinyl acetate phthalate), polymers or copolymers derived from acrylic and/or methacrylic acid esters, zein, waxes (alone or in admixture with fatty alcohols), shellac, hydrogenated vegetable oils, and mixtures thereof. In addition, an inactive intermediate film may be provided between the active agent, for example, a PPI, and the enteric coating to prevent interaction of the active agent with the enteric coating.

In certain embodiments, a composition described herein comprises a buffering agent. In some instances, an active agent may be protected from the stomach's acidic environment and later release in the duodenum or lower gastrointestinal tract through other means, including buffering the active agent, for example a PPI, with a buffering agent, including sodium bicarbonate, sodium carbonate, calcium carbonate, magnesium oxide, magnesium hydroxide, magnesium carbonate, aluminum hydroxide, or combinations thereof. In certain instances, the stomach's acidic environment interacts with an effervescent buffering agent, e.g., a carbonate or bicarbonate (e.g., sodium bicarbonate, sodium carbonate, calcium carbonate, magnesium carbonate), so as to deliver the active agent to the esophagus (e.g., lower esophagus).

In some embodiments of the invention, the composition includes a combination of a corticosteroid and an acid inhibitor, together with an excipient that increases the interaction of the composition with a surface (e.g., a mucosal or epithelial layer) of the gastrointestinal tract (e.g., esophagus). The combination may include, for example, about 0.25 mg to about 20 mg, about 0.25 mg to about 15 mg, about 0.25 mg to about 10 mg, about 0.25 mg to about 5 mg, 250 µg to 3 mg, or 500 µg to 3 mg, or 500 µg to 2 mg, or 1 mg to 3 mg of a corticosteroid, such as budesonide, together with 50 to 300 mg, 100 to 300 mg, 200-300 mg ranitidine. Other embodiments of the invention may include more than one corticosteroid and/or acid inhibitor, for example, a combination of budesonide and fluticasone together with ranitidine, or a combination of budesonide together with ranitidine and omeprazole.

The exact dosage will depend upon the route of administration, the form in which the composition is administered, the subject to be treated, the age, body weight/height of the subject to be treated, and the preference and experience of the attending physician. The optimal concentration of the corticosteroid and/or acid inhibitor in the composition will depend upon the specific active agent used, the characteristics of the patient, and the nature of the inflammation and/or acid reduction for which the treatment is sought. These factors can be determined by those of skill in the medical and pharmaceutical arts in view of the present disclosure.

Similarly, a therapeutically effective dose of acid inhibitor refers to the amount of acid inhibitor that results in a degree of amelioration of symptoms and acid reduction relative to the status of such symptoms prior to treatments. The dosage forms containing effective amounts are within the bounds of routine experimentation, and therefore, well within the scope of the instant invention. Such doses of acid inhibitors may include 1 µg to 10 mg/kg of body weight per day, or for example, in the range of 2.5 µg to 1 mg/kg of body weight per day. In a preferred embodiment, 100 µg-1 mg/kg of body weight per day of an acid inhibitor is administered.

In an illustrative embodiment, a dosage or amount (including a divided dose) of corticosteroid is provided in a composition of sufficient volume to allow any of the compositions disclosed herein to reach the targeted and/or inflamed portion of the gastrointestinal tract, including, e.g., the esophagus, in an effective amount. In some embodiments, the effective amount of the composition delivered to the esophagus is an amount sufficient to coat or at least partially coat the esophagus, and deliver the composition to the affected areas, including by way of example only, the lower esophagus, the esophageal-stomach juncture, the stomach and/or the duodenum. In certain embodiments, a composition described herein as a volume of, for example about 1-50 mL, or for example about 1-40 mL, or for example about 1-30 mL, or for example about 1-25 mL, or for example, about 1-20 mL, or for example about 5-25 mL, or for example about 10-20 mL, or for example about 10 mL, or for example, about 15 mL, or for example, about 20 mL, or for example about 1-15 mL, or for example about 1-10 mL, or for example about 2-8 mL, or for example about 3-7 mL, or for example, about 4-6 mL, or for example, about 5 mL, or for example about 6-14 mL, or for example about 8-12 mL, or for example, about 9-11 mL, or for example, about 10 mL. In more specific embodiments, about 0.25 mg to about 6 mg, about 0.375 mg, about 0.5 mg, about 0.75 mg, about 1 mg, about 1.25 mg, about 1.5 mg, or about 2 mg of corticosteroid (e.g., budesonide) is formulated into a single or unit dose of a pharmaceutical composition described herein, the single or unit dose having a total volume of about 1-20mL, about 10-20 mL, or for example about 10 mL, or for example, about 15 mL, or for example, about 20 mL, or for example about 1-15 mL, or for example about 1-10 mL, or for example about 2-8 mL, or for example about 3-7 mL, or for example, about 4-6 mL, or for example, about 5 mL, or for example about 6-14 mL, or for example about 8-12 mL, or for example, about 9-11 mL, or for example, about 10 mL. As discussed herein, "liquid" encompasses slurries, solutions, suspensions, dispersions or any combination thereof, depending on the solubilities and amounts of the individual components and the vehicles and solvents used. In some embodiments, an appropriate palatable dosage is in a volume sufficient to coat or at least partially coat the esophagus, and in an illustrative embodiment, the volume is sufficient to coat or at least partially coat the esophagus and deliver the corticosteroid to the affected areas, including by way of example only, the lower esophagus, the esophageal-stomach juncture, the stomach, the duodenum and/or within 3 cm of the Z-line. The composition may be delivered, for example, four times a day, three times a day, twice a day, once a day, every other day, three times a week, twice a week, or once a week. The dosage may, for example, be divided into multiple doses throughout the day, or be provided, for example, in four, three, two, or one dose a day. In certain instances, administration more frequent administration (e.g., b.i.d. versus once a day) provides for a shorter overall therapy or a quicker onset of symptom resolution. In one illustrative example, the dose is provided once a day.

In certain embodiments, a dose or composition described herein is administered with food. In some embodiments, a dose or composition described herein is administered without food. In certain embodiments, a dose or composition described herein is administered in a fed or fasted state. In some embodiments, a dose or composition described herein is administered in the morning, in the afternoon, in the evening, at night, or a combination thereof. In one embodiment, the dose is administered at night. In another aspect, the dose is administered about 30 minutes prior to bed, with no food or water given after administration of the compositions herein. In yet another embodiment of the instant invention, the dose is administered prior to bedtime, wherein after administration of the composition, the patient or individual is in a substantially supine position for at least 30 minutes, at least 1 hour, at least 2 hours, at least 4 hours or at least 8 hours.

In some embodiments, provided herein are methods of treating, preventing, or alleviating inflammation or symptoms associated with inflammation of the gastrointestinal tract, e.g., the esophagus, comprising administering to an individual in need thereof a single unit dose of a pharmaceutical composition described herein from a multidose container. In specific embodiments, administering a single unit dose from a multi dose container comprises (1) shaking a multidose container, the multidose container comprising at least one unit dose of a pharmaceutical composition described herein; (2) pouring (or otherwise dispensing) a single unit dose from the multidose container into an administration device (e.g., a device suitable for administering to a human individual, such as a spoon, cup or syringe); and (3) administering the single unit dose to the individual in need thereof. In more specific embodiments, shaking of the multidose container occurs until the fluid therein has a viscosity suitable for pouring (e.g., easy pouring). In some specific embodiments, the process further comprises waiting after pouring the single unit dose and prior to administering the single unit dose to the individual in need thereof. In specific embodiments, the wait time is a time sufficient to allow the viscosity of composition to achieve a desired level, e.g., a viscosity to improve the coating capabilities of the composition. In some embodiments, the wait time is, e.g., about 3 seconds, or more; about 5 seconds, or more; about 10 seconds, or more; about 15 seconds, or more; about 20 seconds, or more; about 25 seconds, or more; about 30 seconds, or more; about 40 seconds, or more; about 45 seconds, or more; about 50 seconds, or more; or about 60 seconds, or more. In other specific embodiments, the composition is administered immediately following pouring the composition into the administration device. In some embodiments, the process comprises shaking the multidose container well.

In other illustrative embodiments of the invention, any of the compositions disclosed herein are provided in the form of a lozenge which may be dissolved in the mouth, thus reaching and coating the esophagus, and thereafter deliver the composition to the affected areas, including by way of example only, the lower esophagus, the esophageal-stomach juncture, the stomach, the duodenum and/or within 3 cm of the Z-line. The lozenge or other similar tablet, capsule, or other solid, would dissolve in the mouth or esophagus to produce a solution that can then coat the esophagus, and thereafter deliver the composition to the affected areas, including by way of example only, the lower esophagus, the esophageal-stomach juncture, the stomach and/or the duodenum. Or, for children, infants or other patients that may have difficulty with a dissolving lozenge, the lozenge may be ground or otherwise dissolved in a small volume of water or other pharmaceutically suitable liquid, for example, reaching a total volume presented in embodiments herein. In other illustrative embodiments of the invention, the compositions disclosed herein are provided in the form of a tablet, a capsule, or, for example a gel capsule, designed for slow release and delivery to the gastrointestinal tract, including the esophagus.

The compositions of the present invention may include pharmaceutically acceptable salts. Pharmaceutically acceptable salts are generally well known to those of ordinary skill in the art and may include, by way of example but not limitation, acetate, atosylate, benzenesulfonate, besylate, benzoate, bicarbonate, bitartrate, bromide, calcium edetate, carnsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, mucate, napsylate, nitrate, pamoate (embonate), pantothenate, phosphate/diphosphate, polygalacturonate, salicylate, stearate, subacetate, succinate, sulfate, tannate, tartrate, or teoclate. Other pharmaceutically acceptable salts may be found in, for example, Remington: The Science and Practice of Pharmacy (20th ed.) Lippincott, Williams & Wilkins (2000). Preferred pharmaceutically acceptable salts include, for example, acetate, benzoate, bromide, carbonate, citrate, gluconate, hydrobromide, hydrochloride, maleate, mesylate, napsylate, pamoate (embonate), phosphate, salicylate, succinate, sulfate, or tartrate. Such salts may be used for the steroid, the PPI, the H₂RA or any combination of the above.

Depending on the specific conditions being treated, the compositions may be formulated into liquid or solid dosage forms and administered systemically or locally. In some embodiments, the agents are delivered, for example, in a timed- or sustained-low release form as is known to those skilled in the art. Techniques for formulation and administration may be found in Remington: The Science and Practice of Pharmacy (20th ed.) Lippincott, Williams & Wilkins (2000).

In addition to the active or actives, various embodiments of the present invention provide for pharmaceutical compositions that contain suitable pharmaceutically acceptable excipients and auxiliaries. For example, in some embodiments, pharmaceutically acceptable excipients and/or auxiliaries are used to formulate the corticosteroids herein disclosed for the practice of the invention into dosages suitable for systemic administration is within the scope of the invention. In some embodiments, the corticosteroid is formulated readily using pharmaceutically acceptable excipients and/or auxiliaries well known in the art into dosages suitable for oral administration. Such excipients and/or auxiliaries enable the compositions of the invention to be formulated as tablets, pills, dragees, capsules, liquids, soft chews, creams, pastes, chewable tablets, gels or gel matrices, syrups, slurries, suspensions, gums, lozenges, and the like, for oral ingestion by a patient to be treated. In certain instances, oral formulations (e.g., suspensions, creams or gel matrices) are formulated such that upon oral administration, an interface layer between the oral formulation (e.g., suspension, cream or gel matrix) and a surface (e.g., a mucosal or epithelial layer) of the gastrointestinal tract (e.g., esophagus). In some instances, an oral formulations (e.g., suspensions, creams or gel matrices) in contact with a surface (e.g., a mucosal or epithelial layer) of the gastrointestinal tract (e.g., esophagus) delivers a corticosteroid to the surface (e.g., a mucosal or epithelial layer) of the gastrointestinal tract (e.g., esophagus)via the interface layer and as the oral formulations (e.g., suspensions, creams or gel matrices) near the interface layer is depleted of corticosteroid, a concentration gradient results. In certain instances, portions of the oral formulations (e.g., suspensions, creams or gel matrices) with high concentrations of corticosteroid relative to the portions of the oral formulations (e.g., suspensions, creams or gel matrices) proximate to the interface layer replenishes corticosteroid in the portion of the oral formulations (e.g., suspensions, creams or gel matrices) proximate to the interface layer. In certain instances, upon oral administration of an oral formulation described herein to an individual, an interface layer is formed between a surface (e.g., a mucosal or epithelial layer) of the gastrointestinal tract (e.g., esophagus) and a mixture of the oral formulation (e.g., chewable tablet) and saliva of the individual.

In certain embodiments, pharmaceutical preparations for oral use are obtained by combining the corticosteroids and/or acid inhibitors with solid excipients, optionally grinding a resulting mixture, and processing the mixture of granules, after adding suitable auxiliaries, if desired, to obtain tablets or dragee cores. Suitable excipients may be, in particular, fillers such as sugars or starches, including dextrose, lactose, sucrose, sucralose, mannitol, or sorbitol; and maize starch, wheat starch, rice starch, or potato starch, or a combination thereof. If desired, disintegrating agents may be added, such as the cross-linked polyvinylpyrrolidone, agar, or alginic acid or a salt thereof such as sodium alginate. Appropriate excipients may also, for example, include those that render the dissolving tablet palatable.

In some embodiments, the pharmaceutical compositions described herein are in liquid form. Appropriate excipients for use in liquid form, appropriate excipients may be used, for example, to render the liquid composition palatable. Excipients may include, for example, either sugars, including dextrose, lactose, sucrose, sucralose, maltodextrin, mannitol, or sorbitol; honey or a combination thereof. Other flavoring or flavor-enhancing agents may also be used.

Liquid suspensions useful herein include, for example, those prepared by adding about 5 to about 25 grams of Splenda®, or about 7 to about 20 grams of Splenda®, or about 5 to about 15 grams of Splenda®, or about 7 to about 15 grams of Splenda®, or about 8 to about 12 grams of Splenda®, or about 10 to about 11 grams of Splenda®, or 1, 2, 3, 4, 5, 6, 7, 8, 9, 10, 11, 12, 13, 14, 15, 16, 17, 18, 18, 19,20,21, 22, 23, 24, or 25 grams of Splenda®, added to 4 ml or 8 ml of a budesonide suspension, such as that obtained from commercially available Pulmicort Respules unit dose, or larger volumes having the same ratios of Splenda® to budesonide.

Any of the compositions or formulations described herein optionally comprise one or more binder, optionally comprise one or more filler, optionally comprise one or more lubricant, optionally comprise one or more solvent, optionally comprise one or more suspension agent, optionally comprise one or more flavoring agent, optionally comprise one or more coloring agent, optionally comprise one or more sweetener, optionally comprise one or more preservative, optionally comprise one or more antioxidant, optionally comprise one or more buffering agent, optionally comprise one or more humectant, optionally comprise one or more chelating agent, optionally comprise one or more surfactant, or combinations thereof.

Preservatives include, by way of non-limiting example, benzalkonium chloride, cetrimide (cetyltrimethylammonium bromide), benzoic acid, benzyl alcohol, methyl-, ethyl-,propyl-and butyl-esters of para-hydroxybenzoic acid, chlorhexidine, chlorobutanol, phenylmercuric acetate, borate and nitrate, potassium sorbate, sodium benzoate, sorbic acid, thiomersal (mercurithiosalicylate), combinations thereof, or the like. Compositions and formulations described herein optionally include any suitable amount of preservative including, by way of non-limiting example, about 0.1 % w/w to about 5% w/w, about 0.1 % w/w to about 3% w/w, about 0.1% w/w to about 1% w/w, about 0.1% w/w to about 0.5% w/w of one or more preservative(s).

Antioxidants include, by way of non-limiting example, ascorbyl palmitate, butylated hydroxyanisole, butylated hydroxytoluene, monothioglycerol, sodium ascorbate, sodium formaldehyde sulfoxylate, sodium metabisulfite, BHT, BHA, sodium bisulfite, vitamin E or a derivative thereof, propyl gallate, edetate (EDTA) (e.g., disodium edetate), Diethylenetriaminepentaacetic acid (DTPA), Triglycollamate (NT), combinations thereof, or the like. Compositions and formulations described herein optionally include any suitable amount of antioxidant including, by way of non-limiting example, about 0.01% w/w to about 0.5% w/w, about 0.01% w/w to about 0.3% w/w, or about 0.01% w/w to about 0.1% w/w one or more antioxidant(s).

Buffering agents include, by way of non-limiting example, citrate buffers (i.e., citric acid and citrate), phosphate buffers, acetate buffers, combinations thereof, or the like. In various embodiments, any suitable amount of buffering agent(s) are utilized and any suitable pH is achieved.

As used herein, "citrate" includes all compounds of Formula I wherein each R is independently selected from an H and a negative charge (e.g., as a salt or as a disassociated salt or acid). In certain embodiments, citrate is selected from, by way of non-limiting example, sodium citrate, citric acid and the like.

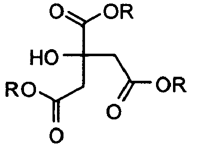

Humectants include, by way of non-limiting example, glycerine, propylene glycol, ethylene glycol, glyceryl triacetate, polyols (e.g., sorbitol, xylitol, maltitol, polydextrose), and the like. Compositions and formulations described herein optionally include any suitable amount of humectant including, by way of non-limiting example, about 0.1% w/w to about 10% w/w, about 1% w/w to about 10% w/w, about 1% to about 8% w/w, or about 5% w/w of a humectant. In certain embodiments, humectants inhibit precipitation and/or crystallization of one or more component of a composition or formulation described herein (e.g., a sweetener, mucoadhesive agent or a viscosity enhancing agent).

Chelating agents include, by way of non-limiting example, edetate (EDTA) (e.g., disodium edetate), Diethylenetriaminepentaacetic acid (DTPA), Triglycollamate (NT), or the like. Compositions and formulations described herein optionally include any suitable amount of chelating agent including, by way of non-limiting example, about 0.01% w/w to about 0.5% w/w, about 0.01% w/w to about 0.3% w/w, or about 0.01% w/w to about 0.1% w/w, or about 0.05% w/w of one or more chelating agent.

As used herein, "edetate" includes all compounds of Formula II wherein each R is independently selected from an H and a negative charge (e.g., as a salt or as a disassociated salt or acid). In certain embodiments, edetate is selected from, by way of non-limiting example, disodium edetate, calcium edetate, ethylenediaminetetraacetic acid and the like.

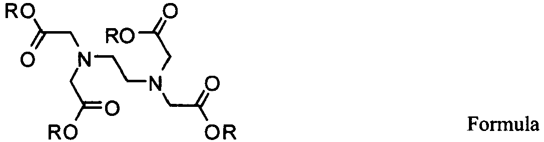

In certain embodiments, sweeteners include, by way of non-limiting example, glycerin, acesulfame potassium (AceK), mono-ammonium glycyrrhizinate (e.g., Magnasweet®), sucrose, lactose, glucose, fructose, arabinose, xylose, ribose, mannose, galactose, dextrose, sorbose, sorbitol, mannitol, maltose, cellobiose, xylitol and the like. In some embodiments, flavoring agents include, by way of non-limiting example, peppermint, orange, bubble gum, wintergreen, grape and cherry. In various embodiments, any amount of sweetener and/or flavoring agent is optionally utilized. In specific embodiments, enough sweetener and/or flavoring agent is utilized to render any composition described herein palatable. In certain embodiments, a composition or formulation described herein comprises dextrose. In some embodiments, the composition or formulation comprises less than 50% w/w, 40% w/w, 30% w/w, 20% w/w, 15% w/w, 10% w/w, 5% w/w, or 3% w/w of dextrose. In certain embodiments, the dextrose is substantially dissolved in a liquid vehicle of the composition or formulation. In certain embodiments, coloring agents include yellow agents (e.g., FD&C 5 and/or 6), red agents (e.g., FD&C Red 40), blue, or the like.

Surfactants include, e.g., anionic, cationic, non-ionic, or zwitterionic surfactants, such as, by way of non-limiting example, polysorbate (e.g., polysorbate 20, polysorbate 60, polysorbate 40, polysorbate 80, polysorbate 81, polysorbate 85, polysorbate 120), bile acids or their salts (e.g., sodium taurocholates, sodium deoxytaurocholates, chenodeoxycholic acid, and ursodeoxycholic acid), nonoxynol or polyoxyethylene glycol fatty acid esters, pluronic or poloxamers such as Pluronic F68, Pluronic L44, Pluronic L101, combinations thereof, or the like. Compositions and formulations described herein optionally include any suitable amount of surfactant including, by way of non-limiting example, about 0.001% w/w to about 0.5% w/w, about 0.001% w/w to about 0.3% w/w, or about 0.001% w/w to about 0.1% w/w of one or more surfactant.

Dragee cores are provided with suitable coatings. In some embodiments, concentrated sugar solutions are used for this purpose, which optionally contain gum arabic, talc, polyvinylpyrrolidone, carbopol gel, polyethylene glycol (PEG), and/or titanium dioxide, lacquer solutions, and suitable organic solvents or solvent mixtures. Dye-stuffs or pigments are optionally added to the tablets or dragee coatings for identification or to characterize different combinations of active corticosteroid doses.

In various embodiments, pharmaceutical preparations that are used orally include push-fit capsules made of gelatin, as well as soft, sealed capsules made of gelatin, and a plasticizer, such as glycerol or sorbitol. In some embodiments, the push-fit capsules contain the active ingredient or ingredients in admixture with a filler, binder, lubricant, stabilizer or a combination thereof. Fillers include, by way of non-limiting example, lactose. Binders include, by way of non-limiting example, starches. Lubricants include, by way of non-limiting example, talc and magnesium stearate. In soft capsules, the corticosteroids may be dissolved or suspended in suitable liquids, such as fatty oils, liquid paraffin, or liquid polyethylene glycols (PEGs). In addition, stabilizers are optionally added.

In one embodiment, the present invention provides for an active agent or agents that have a low bioavailability. Due to the low bioavailability, the corticosteroid and/or acid inhibitors are used in certain embodiments of the invention, the active agent(s) remain in the gastrointestinal tract, for example, in the esophagus. In some embodiments, the low bioavailability results in decreased systemic side effects and complications, allowing patients with chronic conditions to receive treatment for longer periods of time.

In some embodiments, a pharmaceutical composition or dosage form described herein is a suspension or a solution comprising a corticosteroid (e.g., budesonide). In some embodiments, compositions (e.g., suspensions) comprise a certain concentration of corticosteroid (e.g., budesonide) that is dissolved in the liquid medium (e.g., the solvent or liquid vehicle used, such as water, alcohol, aqueous alcohol, or the like). In certain embodiments, the amount of corticosteroid (e.g., budesonide) dissolved in the liquid medium is greater than 4 µg/mL, greater than 5 µg/mL, greater than 10 µg/mL, greater than 15 µg/mL, greater than 20 µg/mL, greater than 21 µg/mL, greater than 22 µg/mL, greater than 23 µg/mL, greater than 24 µg/mL, greater than 25 µg/mL, about 25 µg/mL, greater than 30 µg/mL, about 25 µg/mL to about 80 µg/mL, about 30 µg/mL to about 80 µg/mL, about 30 µg/mL, about 35 µg/mL, about 40 µg/mL, about 45 µg/mL, about 50 µg/mL, about 55 µg/mL, about 60 µg/mL, about 65 µg/mL, or about 70 µg/mL.

In some embodiments, compositions (e.g., suspensions) comprise a certain concentration of budesonide that is dissolved in the liquid medium (e.g., the solvent or liquid vehicle used, such as water, alcohol, aqueous alcohol, or the like). In specific embodiments, the amount of R epimer of the dissolved budesonide (compared to the overall weight of the budesonide) is greater than 28% w/w, greater than 30% w/w, greater than 39% w/w, greater than 40%, about 39-50%, about 40-50%, less than 38% w/w, about 29%-37% w/w, less than 27% w/w, or the like. In some instances, the % epimers are obtained in a composition having an overall % R epimer (compared to overall budesonide) of about 50-55% w/w, or about 53-54% w/w. In certain instances, equilibration of the sample is accomplished once the concentration of the corticosteroid (e.g., budesonide) dissolved in the liquid is substantially stable, e.g., after 2 days, 3 days, 4 days, 5 days, a week, a month, or the like. In specific instances, equilibration of the sample is accomplished after 2 days.

In certain embodiments, the compositions provided herein are prepared utilizing any suitable source of active agents. In some embodiments, corticosteroid (e.g., budesonide) used in the compositions described herein are neat corticosteroid (e.g., budesonide). In some embodiments, the neat corticosteroid (e.g., budesonide) is neat, bulk corticosteroid. In certain embodiments, the neat corticosteroid (e.g., budesonide) is powder corticosteroid (e.g., budesonide). In specific embodiments, the neat corticosteroid (e.g., budesonide) is micronized corticosteroid (e.g., budesonide).

In some embodiments, the corticosteroid is administered in a commercially available formulation. In other embodiments, the corticosteroid is administered in a composition comprising a commercially available formulation of a corticosteroid and formulated as described herein. For example, in some embodiments, the corticosteroid containing composition provided herein comprises a commercially available formulation and an excipient, such as a diluents, a flavoring agent, a mucoadhesive agent, a viscosity enhancing agent, a binder, a filler, a lubricant, a solvent, a suspension agent, a coloring agent, a sweetener, a preservative, an antioxidant, a buffering agent, a humectant, a chelating agent, a surfactant, combinations thereof, or the like. In some embodiments, wherein the corticosteroid is budesonide, the commercially available formulation is Pulmicort Respules® (distributed by AstraZeneca, e.g., as set forth in NDA 20-929, which is hereby incorporated by reference in its entirety). In other embodiments, wherein the corticosteroid is budesonide, the commercially available formulation is Rhinocort Aqua® (distributed by AstraZeneca LP, Wilmington, DE 19850, e.g., as set forth in NDA 20-746, which is, including all supplements, hereby incorporated herein by reference in its entirety). In still other embodiments, wherein the corticosteroid is budesonide, the commercially available formulation is Symbicort® (manufactured by AstraZeneca Dunkerque Production, Dunkerque, France, e.g., as set forth in NDA 21-929, which is, including all supplements, hereby incorporated herein by reference in its entirety). In some embodiments, wherein the corticosteroid is fluticasone, the commercially available formulation is Flonase®. In some embodiments, the ratio of commercially available formulation to the optional diluent is between about 1:0.5 and about 1:100. Diluents include any pharmaceutically acceptable oral diluent including, e.g., powder diluents (such as talc) and liquid diluents (such as water, ethanol and combinations thereof). In certain embodiments, the commercially available formulation is Entocort® (manufactured by AstraZeneca AB, S-151 85 Sodertalje, Sweden, distributed by Prometheus Laboratories Inc, San Diego, CA 92121, as set forth in NDA 21-324, which is, including all supplements. In certain embodiments, Entocort® formulations are dissolved and/or dispersed in an aqueous vehicle. In specific embodiments, the Entocort® formulation is dispersed in a liquid vehicle that has a pH sufficient to remove the enteric coating from the budesonide particles. In other embodiments, the Entocort® formulation is pretreated with a solvent having a pH sufficient to remove the enteric coating from the budesonide particles therein, and the particles are subsequently formulated into a composition described herein.

In certain embodiments, a corticosteroid composition described herein comprises a corticosteroid, a commercially available formulation, and, optionally, one or more additional excipient. In some embodiments, a corticosteroid composition described herein comprises a corticosteroid formulated in a manner similar to a commercial formulation (e.g., lacking one or more of the active ingredients of the formulation), and, optionally, one or more additional excipient. The one or more additional excipients can be utilized to achieve a formulation as described herein. In specific embodiments, the commercially available formulation is Ultra XCID (manufactured by Matrixx Initiatives, Inc., Phoenix, AZ).

In certain embodiments, the corticosteroid containing composition comprises micronized budesonide, disodium edetate, sodium chloride, sodium citrate, citric acid, polysorbate (e.g., polysorbate 80), water, and optionally one or more excipients, wherein the excipients are selected from any of those recited herein. In certain embodiments, the composition comprises about 0.1 mg to about 1.0 mg budesonide/2 mL composition. In some embodiments, the composition comprises about 0.2 mg to about 0.6 mg budesonide/2 mL composition. In specific embodiments, the composition comprises about 0.25 mg/2 mL composition. In other specific embodiments, the composition comprises about 0.5 mg/2 mL composition.

In other embodiments, the corticosteroid containing composition comprises micronized budesonide, microcrystalline cellulose, carboxymethyl cellulose sodium, dextrose anhydrous, polysorbate (e.g., polysorbate 80), disodium edetate, potassium sorbate, water, optionally hydrochloric acid and optionally one or more excipients, wherein the excipients are selected from any of those recited herein. In specific embodiments, the composition has a pH of about 4.5. In some embodiments, the composition comprises about 0.1 mg to about 1.0 mg of budesonide/g composition. In certain embodiments, the composition comprises about 0.3 mg to about 0.6 mg of budesonide/g composition. In specific embodiments, the composition comprises about 0.4 mg or 0.44 mg of budesonide/g composition. In certain specific embodiments, the composition comprises about 3.8 mg/8.6 g composition. In some embodiments, the composition comprises about 0.1 mg to about 1.0 mg of budesonide/mL of composition (about 0.01 to about 0.1% w/w). In certain embodiments, the composition comprises about 0.3 mg to about 0.8 mg of budesonide/mL of composition (about 0.03 to about 0.08% w/w). In specific embodiments, the composition comprises about 0.6 to about 0.7 mg of budesonide/mL of composition (about 0.06 to about 0.07% w/w). In more specific embodiments, the composition comprises about 0.63 mg of budesonide/mL of composition (about 0.063% w/w).

In some embodiments, the corticosteroid containing composition comprises microfine fluticasone propionate, microcrystalline cellulose, carboxymethylcellulose sodium, dextrose, benzalkonium chloride, polysorbate (e.g., polysorbate 80), phenylethylalcohol, and optionally one or more excipients, wherein the excipients are selected from those recited herein. In some embodiments, the composition has a pH of between about 5 and about 7. In certain embodiments, the composition comprises about 20 to about 80 µg fluticasone propionate/mg composition. In some embodiments, the composition comprises about 40 to about 60 µg fluticasone propionate/mg composition. In specific embodiments, the composition comprises about 50 µg fluticasone propionate/mg composition. In some embodiments, the composition comprises about 0.02% w/w benzalkonium sodium and about 0.25% w/w phenylethyl alcohol.

### Disorders

It will be appreciated by those skilled in the art that reference herein to treatment extends to prophylaxis as well as the treatment of inflammation or other symptoms.

Thus, provided herein is a method of treating, preventing or alleviating inflammation associated with GERD in an individual comprising orally administering to said individual any of the compositions described herein. Furthermore, provided herein is a method of treating, preventing or alleviating the symptoms of GERD in an individual comprising orally administering to said individual any of the compositions described herein.

In one embodiment, the present invention embodies a method of treating, preventing or alleviating the symptoms of GERD in an individual comprising orally administering to said individual a composition comprising (i) a corticosteroid; and (ii) an excipient or combination of excipients. In some embodiments, the excipient may increase the interaction of the composition with the esophagus, including excipients that increase the viscosity of the composition or impart a mucoadhesive characteristic to the composition. In other embodiments, the excipient may enhance absorption of the active agents across a surface (e.g., a mucosal or epithelial layer) of the gastrointestinal tract (e.g., esophagus). In yet other embodiments, the excipient may include a combination of a viscosity increasing agent, a mucoadhesive agent or an absorption enhancing agent. In other aspects, the excipient may not impart the characteristic of viscosity increasing, mucoadhesiveness or absorption enhancing to the composition. In some embodiments, the excipient is a binder, filler, lubricant or a combination thereof.

One aspect of the invention provided herein is a method of treating, preventing or alleviating the symptoms of GERD in an individual comprising orally administering to said individual a composition comprising (i) a corticosteroid; and (ii) an H₂RA. In some embodiments, the composition further comprises an excipient or combination of excipients. In some embodiments, the excipient may increase the interaction of the composition with the esophagus, including excipients that increase the viscosity of the composition or impart a mucoadhesive characteristic to the composition. In other embodiments, the excipient may enhance absorption of the active agents across a surface (e.g., a mucosal or epithelial layer) of the gastrointestinal tract (e.g., esophagus). In yet other embodiments, the excipient may include a combination of a viscosity increasing agent, a mucoadhesive agent or an absorption enhancing agent. In other aspects, the excipient may not impart the characteristic of viscosity increasing, mucoadhesiveness or absorption enhancing to the composition. In some embodiments, the composition also comprises an excipient including, e.g., a binder, filler, lubricant or a combination thereof.

Another aspect of the invention provided herein is a method of preventing or alleviating esophageal inflammation in an individual comprising orally administering to said individual a composition comprising (i) a corticosteroid, and (ii) a PPI. In some embodiments, the composition further comprises an excipient or combination of excipients. In some embodiments, the excipient may increase the interaction of the composition with the esophagus, including excipients that increase the viscosity of the composition or impart a mucoadhesive characteristic to the composition. In other embodiments, the excipient may enhance absorption of the active agents across a surface (e.g., a mucosal or epithelial layer) of the gastrointestinal tract (e.g., esophagus). In yet other embodiments, the excipient may include a combination of a viscosity increasing agent, a mucoadhesive agent or an absorption enhancing agent. In other aspects, the excipient may not impart the characteristic of viscosity increasing, mucoadhesiveness or absorption enhancing to the composition. In some embodiments, the composition also comprises an excipient including, e.g., a binder, filler, lubricant or a combination thereof.

Yet another aspect of the invention provided herein is a method of preventing or alleviating esophageal inflammation in an individual comprising orally administering to said individual a composition comprising (i) a corticosteroid, (ii) an H₂RA, and (iii) a PPI. In some embodiments, the composition further comprises an excipient or combination of excipients. In some embodiments, the excipient may increase the interaction of the composition with the esophagus, including excipients that increase the viscosity of the composition or impart a mucoadhesive characteristic to the composition. In other embodiments, the excipient may enhance absorption of the active agents across a surface (e.g., a mucosal or epithelial layer) of the gastrointestinal tract (e.g., esophagus). In yet other embodiments, the excipient may include a combination of a viscosity increasing agent, a mucoadhesive agent or an absorption enhancing agent. In other aspects, the excipient may not impart the characteristic of viscosity increasing, mucoadhesiveness or absorption enhancing to the composition. In some embodiments, the composition also comprises an excipient including, e.g., a binder, filler, lubricant or a combination thereof.

Viscosity may be, for example, measured at room temperature, at about 20-25 degrees Celsius, or at about 37 degrees Celsius to mimic body temperature. In various embodiments of the present invention, the viscosity of the composition described herein is any viscosity suitable for delivery of the corticosteroid to the targeted and/or inflamed portion of the gastrointestinal tract. In some embodiments, the viscosity of the composition is at least about 1 mPa.s at least about 2 mPa.s, at least about 3 mPa.s, at least about 5 mPa.s, at least about 10 mPa.s, at least about 15 mPa.s, at least about 20 mPa.s, at least about 25 mPa.s, at least about 30 mPa.s, at least about 35 mPa.s, at least about 40 mPa.s, at least about 50 mPa.s, at least about 200 mPa.s, or at least about 225 mPa.s. In some embodiments, the viscosity of the composition is at least about 100 mPa.s. In certain embodiments, the viscosity of the composition, measured at 25 degrees Celsius, is about 50 mPa.s to about 250,000 mPa.s, about 50 mPa.s to about 70,000 mPa.s, about 50 mPa.s to about 25,000 mPa.s, about 50 mPa.s to about 10,000 mPa.s, about 50 mPa.s to about 3,000 mPa.s, or about 50 mPa.s to about 2,000 mPa.s. In one aspect, the viscosity of the composition, as measured at 25 degrees Celsius, is from about 25 mPa.s to about 800 mPa.s, about 50 mPa.s to about 800, or about 300 mPa.s to about 800 mPa.s (e.g., measured by a Brookfield viscometer). In another aspect, the viscosity of the composition may range from about 100 mPa.s to about 200 mPa.s, about 200 mPa.s to about 300 mPa.s, about 250 mPa.s to about 600 mPa.s or about 400 mPa.s to about 600 mPa.s. In specific embodiments, the viscosity of the formulation is about 30 mPa.s, about 100 mPa.s, about 200 mPa.s, about 300 mPa.s, about 400 mPa.s, about 500 mPa.s, or about 250,000 mPa.s (e.g., as measured with a Brookfield viscometer at 25 degrees Celsius equipped with an ultra low adapter).

The unit centipoise is not a SI-unit. The unit mPa.s is the corresponding SI-unit. In some embodiments, the viscosity of the composition is measured at room temperature (about 25 degrees C) with a shear rate of about 13.2 sec⁻¹. In certain embodiments, provided herein is a composition having a viscosity under such conditions that is at least about 2 centipoise (cP), at least about 3 cP, at least about 5 cP, at least about 10 cP, at least about 15 cP, at least about 20 cP, at least about 25 cP, at least about 30 cP, at least about 35 cP, at least about 40 cP, at least about 50 cP, at least about 200 cP, at least about 225 cP, at least about 250 cP, at least about 300 cP, or at least about 400 cP. In some embodiments, the viscosity of the composition under such conditions is about 50 cP to about 250,000 cP, about 50 cP to about 70,000 cP, about 50 cP to about 25,000 cP, about 50 cP to about 10,000 cP, about 50 cP to about 3,000 cP, about 50 cP to about 2,000 cP, about 250 cP to about 250,000 cP, about 250 cP to about 70,000 cP, about 250 cP to about 25,000 cP, about 250 cP to about 10,000 cP, about 250 cP to about 3,000 cP, or about 250 cP to about 2,000 cP. In one aspect, the viscosity of the composition, as measured at 25 degrees Celsius, is from about 2 centipoise (cP) to about 800 cP, about 25 cP to about 800, about 50 cP to about 800, or about 300 cP to about 800 cP (e.g., measured by a Brookfield viscometer). In another aspect, the viscosity of the composition under such conditions may range from about 100 cP to about 200 cP, about 200 cP to about 300 cP, about 250 cP to about 600 cP or about 400 cP to about 600 cP. In specific embodiments, the viscosity of the formulation measured under such conditions is about 30 cP, about 40 cP, about 100 cP, about 200 cP, about 300 cP, about 400 cP, about 500 cP, or about 250,000 cP.

The unit centipoise is not a SI-unit. The unit mPa.s is the corresponding SI-unit. In some embodiments, the viscosity of the composition is measured at room temperature (about 25 degrees C) with a shear rate of about 15 sec⁻¹ (e.g., with a gap between the spindle and the sample chamber wall of about 6 mm or greater). In certain embodiments, provided herein is a composition having a viscosity under such conditions that is at least about 2 centipoise (cP), at least about 3 cP, at least about 5 cP, at least about 10 cP, at least about 15 cP, at least about 20 cP, at least about 25 cP, at least about 50 cP, at least about 100 cP, at least about 150 centipoise (cP), at least about 160 cP, at least about 170 cP, at least about 180 cP, at least about 190 cP, or at least about 200 cP. In some embodiments, the viscosity of the composition under such conditions is about 150 cP to about 250,000 cP, 160 cP to about 250,000 cP, 170 cP to about 250,000 cP, 180 cP to about 250,000 cP, or 190 cP to about 250,000 cP.

In certain embodiments of the invention, the viscosity of the composition is about that of a suspension prepared by adding about 5 to about 15 grams of Splenda® to 4 ml of water, wherein the viscosity is measured at 25 degrees Celsius.

In other embodiments of the invention, the viscosity of the composition is about that of a suspension prepared by adding about 10 to about 12 grams of Splenda® to 4 ml of water, wherein the viscosity is measured at 25 degrees Celsius.

In any of such methods, a viscosity-enhancing excipient can be, for example acacia (gum arabic), agar, aluminum magnesium silicate, sodium alginate, sodium stearate, bladderwrack, bentonite, carbomer, carrageenan, Carbopol, cellulose, microcrystalline cellulose, ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500) gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose, sodium carboxymethyl-cellulose (CMC), silicon dioxide, polyvinylpyrrolidone (PVP: povidone), Splenda® (dextrose, maltodextrin and sucralose) or combinations thereof. In certain embodiments, a viscosity-increasing excipient that may be used is Splenda®. In specific embodiments, the viscosity-enhancing excipient is a combination of MCC and CMC (e.g., Avicel RC-591).

In any such methods of the above, the excipients that impart mucoadhesive characteristics to a composition may include, but are not limited to, at least one soluble polyvinylpyrrolidone polymer (PVP); a water-swellable, but water-insoluble, fibrous, cross-linked carboxy-functional polymer, a crosslinked poly(acrylic acid) (e.g. Carbopol 947P), a carbomer homopolymer, a carbomer copolymer, a hydrophilic polysaccharide gum, maltodextrin, a cross-linked alignate gum gel, a water-dispersible polycarboxylated vinyl polymer, at least two particulate components selected from the group consisting of titanium dioxide, silicon dioxide, and clay, or a mixture thereof.

In any such methods of the above, the excipient that enhances absorption of the composition through a surface (e.g., a mucosal or epithelial layer) of the gastrointestinal tract (e.g., esophagus), may include, but are not limited to, acylcarnitines, surfactants, sodium lauryl sulfate, saponins, bile salts or bile acids including but not limited to cholanic acid, chilic acid, deoxycholic acid, glycocholic acid, tautocholic acid, chenodeoxycholic acid, lithocholic acid, ursocholic acid, ursodeoxycholic acid, isourosde oxycholic acid, lagodeoxycholic acid, glycodeoxycholic acid, glycochenodeoxycholic acid, dehydrocholic acid, hyocholic acid, hyodeoxycholic acid, or combinations thereof, dihydrofusidates, fatty acid derivatives, chitosan, carbopol, cellulosic agents, sterols, including but not limited to alcohols structurally related to steroids, including but not limited to cholestanol, coprostanol, cholesterol, epicholesterol, ergosterol, ergocalciferol, or combinations thereof, starch, dextran, cyclodextrin, or combinations thereof.

In one aspect, a patient is administered a topical corticosteroid such as, for example, budesonide or fluticasone.

H2RAs of the present invention include, but are not limited to, cimetidine, ranitidine, ebrotidine, pabutidine, lafutidine, loxtidine or famotidine. In one non-limiting example, the H2RA is ranitidine.

PPIs of the present invention include, but are not limited to, omeprazole, hydroxyomeprazole, esomeprazole, tenatoprazole, lansoprazole, pantoprazole, rabeprazole, dontoprazole, habeprazole, perprazole, ransoprazole, pariprazole or leminoprazole. In one non-limiting example, the PPI is omeprazole.

Patients to be treated with compositions described herein include those that have been diagnosed with GERD. In some embodiments, the patient suffering from GERD is suffering from NERD. In other embodiments, the patient suffering from GERD is suffering from erosive esophagitis (EE). In some embodiments, the patient suffering from GERD is suffering from Barrett's Esophagus. In still other embodiments, the patient suffering from GERD is suffering from Barret's Esophagus. A patient can be an adult, a child or an infant. In one aspect, a patient is a child less than 16 years old, less than 12 years old, less than 8 years old, less than 6 years old, less than 4 years old or less than 2 years old. In one aspect, a patient is an infant less than one year old, less than 6 months old or less than 3 months old.

A composition of the invention disclosed herein may be in a unit dose formulation for oral administration of a patient. In one aspect, a H2RA is present in the unit dose in an amount of between about 1 mg and about 1g. In some embodiments, the amount of H2RA present in a unit dose is between about 1 mg and about 500 mg, between about 2.5 mg and about 250 mg, or between about 5 mg and about 100 mg. In some embodiments, a PPI is present in the unit dose in an amount of between about 1 mg and about 1.5 g. In certain embodiments, a PPI is present in a unit dose in the amount of between about 1 mg and about 600 mg. In yet another aspect, about 0.1 mg to about 20 mg, about 0.3 mg to about 4 mg, about 0.01 mg to about 20 mg, about 0.01 mg to about 15 mg, or from about 0.25 mg to about 5 mg (e.g., about 1-2 mg/day or about 2-3 mg/day) corticosteroid per day is administered to said individual. In some embodiments, the corticosteroid is present in a unit dose in an amount of between about 0.25 mg and about 5 mg. In some embodiments, the amount of corticosteroid administered daily or in a unit dose is between about 0.5 mg and about 3 mg. In other embodiments, the amount of corticosteroid present in a unit dose or administered daily is between about 1 and about 3 mg, or between about 1 and about 2 mg, or between about 2 and about 3 mg. In other aspects, the unit dose formulation includes a combination of a corticosteroid and an acid inhibitor, including but not limited to an H2RA and/or a PPI.

In certain aspects, about 0.01 mg to about 20 mg, about 0.01 mg to about 15 mg, or about 0.25 mg to about 5 mg (e.g., about 0.1 to about 5 mg, about 0.25 to about 2.5 mg, about 0.3 mg to about 2 mg, about 0.5 mg to about 1 mg, about 0.7 mg to about 1.5 mg, about 0.375 mg, about 0.75 mg, about 1 mg, about 1.25 mg, about 1.5 mg or about 2 mg) corticosteroid per day is administered to a patient. In some embodiments, the corticosteroid is present in a unit dose in an amount of between about 0.25 mg and about 5 mg. In some embodiments, the amount of corticosteroid administered daily or in a unit dose is between about 0.5 mg and about 3 mg. In other embodiments, the amount of corticosteroid present in a unit dose or administered daily is between about 1 and about 3 mg, or between about 1 and about 2 mg, or between about 2 and about 3 mg.

In some embodiments, the corticosteroid is present in a pharmaceutical composition described herein in any effective amount. In some embodiments, an effective amount is an amount sufficient to reduce inflammation or symptoms of inflammation associated with an inflammatory disease or condition of the gastrointestinal tract (e.g., the esophagus) as compared to the level of inflammation or symptoms of inflammation associated with an inflammatory disease prior to administration of the effective amount. In certain embodiments, effective amount is an amount sufficient to maintain a reduction in inflammation or symptoms of inflammation achieved in any manner including, but not limited to, by the administration of an effective amount sufficient to achieve such a reduction. In some embodiments, the effective amount is about 0.05 mg to about 20 mg, about 0.05 mg to about 15 mg, about 0.05 mg to about 10 mg, about 0.05 mg to about 7.5 mg, about 0.05 mg to about 5 mg, about 0.25 mg to about 3 mg, about 0.25 mg to about 2.5 mg, about 0.5 mg to about 3 mg, about 0.5 mg to about 2 mg, about 0.5 mg to about 0.1 mg, about 0.5 mg to about 5 mg, about 0.5 mg to about 4 mg, about 1 mg to about 4 mg, about 1 mg to about 3 mg, about 2 mg to about 3 mg, or about 2 mg to about 4 mg. In specific embodiments, the effective amount of corticosteroid is about 0.05 mg, about 0.1 mg., about 0.15 mg., about 0.25 mg., about 0.3 mg., about 0.35 mg, about 0.4 mg, about 0.37 mg, about 0.375 mg, about 0.7 mg, about 0.8 mg, about 0.75 mg, about 1 mg, about 1.2 mg, about 1.25 mg, about 1.3 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, or about 7.5 mg or more. In certain embodiments, the corticosteroid is present in a pharmaceutical composition at a concentration of about 0.01 mg/mL to about 2 mg/mL of composition. In specific embodiments, the corticosteroid is present in a pharmaceutical composition at a concentration of about 0.01 mg/mL to about 1.5 mg/mL, about 0.02 mg/mL to about 1.5 mg/mL, about 0.04 mg/mL to about 1.5 mg/mL, about 0.03 mg/mL to about 1.5 mg/mL, about 0.05 mg/mL to about 1.5 mg/mL, or about 0.07 mg/mL to about 1.5 mg/mL. In more specific embodiments, the corticosteroid is present in a pharmaceutical composition at a concentration of about 0.07 mg/mL to about 1 mg/mL.

In some embodiments, the corticosteroid is selected from, by way of non-limiting example, budesonide, fluticasone propionate and combinations thereof. In specific embodiments, corticosteroid is present in the composition in an amount of about 0.01 mg/mL to about 3 mg/mL, about 0.01 mg/mL to about 2 mg/mL, about 0.01 mg/mL to about 1.5 mg/mL, about 0.07 mg/mL to about 1.5 mg/mL, or about 0.07 mg/mL to about 1 mg/mL. In more specific embodiments, budesonide is present in an amount of about 0.01 mg/mL to about 3 mg/mL, about 0.01 mg/mL to about 1.5 mg/mL, or about 0.07 mg/mL to about 1 mg/mL. In other specific embodiments, fluticasone propionate is present in an amount of about 0.005 mg/mL to about 1.5 mg/mL, or about 0.01 mg/mL to about 1 mg/mL.

In some embodiments, the volume of a composition or dose of a composition described herein is an amount sufficient to substantially coat (e.g., at least 50%, at least 60%, at least 70%, at least 80%, at least 90%, at least 95%, at least 98% or at least 99% of) the length of the esophagus of an individual to whom the composition is administered. In certain embodiments, the volume of a composition or a dose of a composition described herein is about 0.05 mL/cm esophageal length to about 1 mL/cm esophageal length, about 0.1 mL/cm esophageal length to about 0.8 mL/cm esophageal length, about 0.2 mL/cm esophageal length to about 0.6 mL/cm esophageal length, or about 0.3 mL/cm esophageal length to about 0.5 mL/cm esophageal length, wherein the esophageal length is the esophageal length of the individual to whom the composition is administered. In some embodiments, the volume of a composition or dose of a composition described herein is based on the esophageal length of an individual (e.g., male, female, or both) that is in the 50^{th} percentile of height for their age. Therefore, in some embodiments, the volume of a composition or dose of a composition described herein is about 0.05 mL/cm esophageal length to about 1 mL/cm esophageal length, about 0.1 mL/cm esophageal length to about 0.8 mL/cm esophageal length, about 0.2 mL/cm esophageal length to about 0.6 mL/cm esophageal length, about 0.3 mL/cm esophageal length to about 0.5 mL/cm esophageal length, about 0.32 mL/cm esophageal length to about 0.41 mL/cm esophageal length, or about 0.3 mL/cm esophageal length to about 0.46 mL/cm esophageal length, wherein the esophageal length is the esophageal length of an individual having a height in the 50^{th} percentile for the age of the individual to whom the composition is administered. In certain instances, esophageal length is the actual esophageal length of the individual or is calculated based on the equation: esophageal length = 1.048(cm) + (0.167*height(cm)). In certain instances, for example, the 50^{th} percentile height (CDC 2000) for male children age 2 is 87 cm, age 3 is 95 cm, age 4 is 102 cm, age 5 is 109 cm, age 6 is 115 cm, age 7 is 122 cm, age 8 is 128 cm, age 9 is 134 cm, age 10 is 139 cm, age 11 is 144 cm, age 12 is 149 cm, age 13 is 156 cm, age 14 is 164 cm, age 15 is 170 cm, age 16 is 174 cm, age 17 is 175 cm, and age 18 is 176 cm.

Furthermore, in certain embodiments, the amount of a therapeutic agent (e.g., a corticosteroid such as budesonide) in a composition or a dose of a composition described herein is about 0.005 mg/cm esophageal length to about 0.3 mg/cm esophageal length, about 0.008 mg/cm esophageal length to about 0.2 mg/cm esophageal length, about 0.01 mg/cm esophageal length to about 0.15 mg/cm esophageal length, or about 0.015 mg/cm esophageal length to about 0.1 mg/cm esophageal length, wherein the esophageal length is the esophageal length of the individual to whom the composition is administered. In some embodiments, the volume of a composition or dose of a composition described herein is based on the esophageal length of an individual (e.g., male, female, or both) that is in the 50^{th} percentile of height for their age. Therefore, in some embodiments, the amount of a therapeutic agent (e.g., a corticosteroid such as budesonide) in a composition or dose of a composition described herein is about 0.005 mg/cm esophageal length to about 0.3 mg/cm esophageal length, about 0.008 mg/cm esophageal length to about 0.2 mg/cm esophageal length, about 0.01 mg/cm esophageal length to about 0.15 mg/cm esophageal length, or about 0.015 mg/cm esophageal length to about 0.1 mg/cm esophageal length, wherein the esophageal length is the esophageal length of an individual having a height in the 50^{th} percentile for the age of the individual to whom the composition is administered.

In some embodiments, any pharmaceutical composition or dose of a pharmaceutical composition described herein is provided or administered in a volume sufficient to provide a bolus when orally administered to an individual. In certain embodiments, the composition has a volume that does not systemically deliver excessive amounts of the active agent. In some embodiments, the pharmaceutical composition or dose is provided in a volume sufficient to provide a bolus when administered to an individual, wherein the size of the bolus at the distal end of the esophagus (e.g., the size of the bolus prior, e.g., immediately prior, to entering or passing the lower esophageal sphincter) is less than 90%, less than 85%, less than 80%, less than 75%, less than 70%, less than 65%, less than 60%, less than 55%, less than 50%, less than 45%, less than 40%, less than 35%, less than 30%, less than 25%, less than 20%, less than 15%, less than 10% or less than 5% of size of the bolus that entered the esophagus (e.g., the size of the bolus after, e.g., immediately after, passing the upper esophageal sphincter). In some embodiments, the size of the bolus is determined as a measure of diameter or of volume. In certain embodiments, diameter of the sphincter can be determined using gamma scintigraphy techniques. In specific embodiments, the volume of the composition or dose is adjusted given the length and/or diameter of the esophagus of the individual to whom the composition or dose is administered.

In some embodiments, provided herein is a multiple unit container comprising about 2 to about 180, about 10 to about 60, about 14, or about 30 unit doses of any pharmaceutical composition described herein. In more specific embodiments, each dose comprises about 1 mL to about 25 mL, about 1 mL to about 20 mL, about 7 mL to about 25 mL, about 10 to about 20 mL, about 15 mL, about 20 mL, about 3 to about 7 mL, about 5 mL, about 8 mL to about 12 mL, or about 10 mL. In still more specific embodiments, each dose comprises about 0.1 to about 20 mg, about 0.1 to about 10 mg, about 0.1 to about 7.5 mg, about 0.1 to about 5 mg, about 0.3 to about 4 mg, about 0.25 to about 2.5 mg, about 0.3 mg to about 2 mg, about 0.5 mg to about 1 mg, about 0.7 mg to about 1.5 mg, about 0.375 mg, about 0.75 mg, about 1 mg, about 1.25 mg, about 1.5 mg or about 2 mg of corticosteroid. In certain embodiments, provided herein is a multiple unit container comprising about 10 mL to about 1500 mL, about 50 mL to about 600 mL, about 150 mL, about 300 mL, about 600 mL, or about 1,200 mL of any pharmaceutical composition described herein. In specific embodiments, the multidose container comprises about 330 mL or about 55 mL of a composition described herein. In some embodiments, a kit provided herein comprises any multidose container as described herein, a pharmaceutical composition as described herein (e.g., in a volume described), and a delivery or metered device (e.g., a syringe, a cup, a spoon, or the like). In specific embodiments, the delivery device is incorporated into the container (e.g., a nebulizer, an aerosolizer, a pump, or the like). In certain embodiments, the pharmaceutical composition contained within any of the multiple unit containers described herein is physically and chemically stable. The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents.

Unless defined otherwise, all technical and scientific terms used herein have the same meanings as commonly understood by one of ordinary skill in the art to which this invention belongs. Although any methods and systems similar or equivalent to those described herein can be used in the practice or testing of the present invention, the methods, devices, and materials are now described.

The invention illustratively described herein suitably may be practiced in the absence of any element(s) not specifically disclosed herein. Thus, for example, in each instance herein any of the terms "comprising", "consisting essentially of", and "consisting of" may be replaced with either of the other two terms. Thus, the terms and expressions which have been employed are used as terms of description and not of limitation, equivalents of the features shown and described, or portions thereof, are not excluded, and it is recognized that various modifications are possible within the scope of the invention.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 1:

This example details the efficacy and safety of once daily and twice daily use of budesonide in inducing and maintaining remission of disease activity in individuals (children and/or adults) with GERD. Doses of 0-1 mg, 1-2 mg, 2-3 mg, 3-4 mg, 4-5 mg, and/or 5-6 mg per dose are administered once a day or b.i.d. in volumes of 3, 5, 7, 10, 12, 15, or 17.5 mL. A number of individuals (e.g., 20 per budesonide dose frequency, amount, and volume) are evaluated to determine the symptoms prior to therapy, during therapy and following therapy. Administration is conducted for 7 days, 14 days, and 28 days. Primary Outcome Measures include complete resolution of heartburn and regurgitation (e.g., no more than one day with either mild heartburn or regurgitation over the seven days prior to the assessment time-point). Secondary Outcome Measures include: Number of days with heartburn (daytime and night-time); Number of days with regurgitation (daytime and night-time); Number of heartburn and regurgitation-free days (24hrs); Composite score of heartburn and regurgitation frequency and severity; Time to resolution of symptoms of heartburn/regurgitation; Severity of additional GERD symptoms; Quality of Life (assessed using PAGI-QOL to PGIC (Patient Global Impression of Change); Complete resolution of heartburn; Complete resolution of regurgitation; Average severity of heartburn (daytime and night-time); Average severity of regurgitation (daytime and night-time). These symptoms are scored (e.g., assigning a 3 to the most severe symptoms, a 2 to moderate symptoms, a 1 to mild symptoms, and a 0 to a lack of symptoms) and utilized to determine the efficacy of the treatment.

### References

1. Riddell, R H. The Biopsy Diagnosis of Gastroesophageal Reflux Disease, 'Carditis,' and Barrett's Esophagus, and Sequelae of Therapy. The American Journal of Surgical Pathology (1996) 20(S1):S31-S50.
2. Fass, R et al. Gastroesophageal Reflux Disease - Should We Adopt a New Conceptual Framework? The American Journal of Gastroenterology (2002) 97(8):1901-1909.
3. Lembo, T et al. Inflammation of the gastro-oesophageal junction (carditis) in patients with sympomatic gastro-oesophageal reflux disease: a prospective study. Gut (1999) 45:484-488.
4. Haggit, R C. Histophathology of Reflux - Induced Esophageal and Supraesophageal Injuries. The American Journal of Medicine (2000) 108(4A): 109S-111S.

## Claims

1. A composition comprising a therapeutically effective amount of topical corticosteroid for use in treating or alleviating the symptoms of or inflammation associated with gastroesophageal reflux disease (GERD) in an individual, wherein the topical corticosteroid is budesonide, fluticasone, mometasone furoate, ciclesonide, beclomethasone, desonide, or a pharmaceutically acceptable ester thereof, or a combination thereof, by topical administration to the esophagus of the individual.

2. The composition for use of claim 1, wherein the gastroesophageal reflux disease is nonerosive reflux disease (NERD) or erosive esophagitis (EE).

3. The composition for use of claim 1, wherein 100 µg/day to 20 mg/day of the corticosteroid is administered to the individual.

4. The composition for use of claim 3, wherein between 250 µg/day and 5 mg/day of the corticosteroid is administered to the individual.

5. The composition for use of claim 1, wherein the gastroesophageal reflux disease (GERD) is refractory to an acid inhibitor.

6. The composition for use of claim 1, further comprising administering a therapeutically effective amount of an H₂RA, a proton pump inhibitor (PPI), or a combination thereof to said individual.

7. The composition for use of claim 6, wherein the H₂RA is selected from cimetidine, famotidine, nizatidine, and ranitidine.

8. The composition for use of claim 6, wherein the H₂RA is administered in an amount of between 1 mg and 500 mg.

9. The composition for use of claim 6, wherein the proton pump inhibitor is selected from omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, S-tenatoprazole-Na, and dexlansoprazole.

10. The composition for use of claim 6, wherein the proton pump inhibitor is administered in an amount of between 1 mg and 600 mg.

11. The composition for use of claim 1, comprising the corticosteroid and at least one excipient that increases the interaction of the corticodteroid with the individual's esophagus.

12. The composition for use of claim 11, wherein the viscosity of the composition is of 90 mPa.s to 600 mPa.s, wherein the viscosity is measured at 25 degrees Celsius and a shear rate of about 13.2 sec⁻¹.

13. The composition for use of claim 1 or 12, wherein the excipient is a viscosity enhancer, a mucoadhesive agent, an absorption enhancing agent, or a combination thereof.

14. The composition for use of claim 11, wherein the excipient is selected from acacia (gum arabic), agar, aluminum magnesium silicate, sodium alginate, sodium stearate, bladderwrack, bentonite, carbomer, carrageenan, Carbopol, cellulose, microcrystalline cellulose (MCC), ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), a mixture of dextrose, maltodextrin and sucralose, and combinations thereof.

15. The composition for use of claim 14, wherein the excipient is a CMC/MCC combination having a mixed weight ratio of about 11/89.

16. The composition for use of claim 1, wherein the corticosteroid is administered in a unit dose formulation for oral administration.

## Patentansprüche

1. Zusammensetzung, umfassend eine therapeutisch wirksamen Menge eines topischen Kortikosteroids zur Verwendung in der Behandlung oder Linderung der Symptome oder von Entzündung, die mit der gastroösophagealen Refluxkrankheit (GERD) in einem Individuum assoziiert sind/ist, wobei es sich bei dem topischen Kortikosteroid um Budesonid, Fluticason, Mometasonfuroat, Ciclesonid, Beclomethason, Desonid oder einen pharmazeutisch unbedenklichen Ester davon, oder um eine Kombination davon, handelt, mittels topischer Verabreichung an den Ösophagus des Individuums.

2. Zusammensetzung zur Verwendung nach Anspruch 1, wobei es sich bei der gastroösophagealen Refluxkrankheit um nicht-erosive Refluxkrankheit (NERD) oder erosive Ösophagitis (ERD) handelt.

3. Zusammensetzung zur Verwendung nach Anspruch 1, wobei dem Individuum 100 µg/Tag bis 20 mg/Tag des Kortikosteroids verabreicht werden.

4. Zusammensetzung zur Verwendung nach Anspruch 3, wobei dem Individuum zwischen 250 µg/Tag und 5 mg/Tag des Kortikosteroids verabreicht werden.

5. Zusammensetzung zur Verwendung nach Anspruch 1, wobei die gastroösophageale Refluxkrankheit (GERD) refraktär auf einem Säurehemmer ist.

6. Zusammensetzung zur Verwendung nach Anspruch 1, weiterhin umfassend die Verabreichung einer therapeutisch wirksamen Menge eines H₂RA, eines Protonenpumpenhemmers (PPI) oder einer Kombination davon an das Individuum.

7. Zusammensetzung zur Verwendung nach Anspruch 6, wobei der H₂RA aus Cimetidin, Famotidin, Nizatidin und Ranitidin ausgewählt ist.

8. Zusammensetzung zur Verwendung nach Anspruch 6, wobei der H₂RA in einer Menge zwischen 1 mg und 500 mg verabreicht wird.

9. Zusammensetzung zur Verwendung nach Anspruch 6, wobei der Protonenpumpenhemmer aus Omeprazol, Hydroxyomeprazol, Esomeprazol, Tenatoprazol, Lansoprazol, Pantoprazol, Rabeprazol, Dontoprazol, Habeprazol, Perprazol, Ransoprazol, Pariprazol, Leminoprazol, S-Tenatoprazol-Na und Dexlansoprazol ausgewählt ist.

10. Zusammensetzung zur Verwendung nach Anspruch 6, wobei der Protonenpumpenhemmer in einer Menge von zwischen 1 mg und 600 mg verabreicht wird.

11. Zusammensetzung zur Verwendung nach Anspruch 1, umfassend das Kortikosteroid und mindestens einen Grundstoff, der die Wechselwirkung des Kortikosteroids mit dem Ösophagus des Individuums erhöht.

12. Zusammensetzung zur Verwendung nach Anspruch 11, wobei die Viskosität der Zusammensetzung 90 mPa.s bis 600 mPa.s beträgt, wobei die Viskosität bei 25°C und einer Scherrate von ungefähr 13,2 sec⁻¹ gemessen wird.

13. Zusammensetzung zur Verwendung nach Anspruch 1 oder 12, wobei es sich bei dem Grundstoff um einen Viskositätsverstärker, ein Mukoadhäsivum, einen Resorptionsverstärker oder eine Kombination davon handelt.

14. Zusammensetzung zur Verwendung nach Anspruch 11, wobei der Grundstoff aus Gummi arabicum, Agar, Aluminiummagnesiumsilikat, Natriumalginat, Natriumstearat, Blasentang, Bentonit, Carbomer, Carrageenan, Carbopol, Cellulose, mikrokristalliner Cellulose (MCC), Ceratonia, Chondrus, Dextrose, Furcellaran, Gelatine, Ghatti-Gummi, Guar-Gummi, Hektorit, Laktose, Saccharose, Maltodextrin, Mannit, Sorbit, Honig, Maisstärke, Weizenstärke, Reisstärke, Kartoffelstärke, Gelatine, Sterculia-Gumi, Xanthum-Gummi, Polyethylenglykol (z.B. PEG 200-4500), Tragantgummi, Ethylcellulose, Ethylhydroxyethylcellulose, Ethylmethylcellulose, Methylcellulose, Hydroxyethylcellulose, Hydroxyethylmethylcellulose, Hydroxypropylcellulose, Poly(hydroxyethylmethacrylat), Oxypolygelatine, Pectin, Polygelin, Povidon, Propylencarbonat, Methylvinylether/Maleinsäureanhydridcopolymer (PVM/MA), Poly(methoxyethylmethacrylat), Poly(methoxyethoxyethylmethacrylat), Hydroxypropylcellulose, Hydroxypropylmethylcellulose (HPMC), Natriumcarboxymethylcellulose (CMC), Siliziumdioxid, Polyvinylpyrrolidon (PVP: Povidon), einer Mischung aus Dextrose, Maltodextrin und Sucralose, sowie Kombinationen davon, ausgewählt ist.

15. Zusammensetzung zur Verwendung nach Anspruch 14, wobei es sich bei dem Grundstoff um eine CMC/MCC-Kombination mit einem Mischungsgewichtsverhältnis von ungefähr 11/89 handelt.

16. Zusammensetzung zur Verwendung nach Anspruch 1, wobei das Kortikosteroid in einer Einheitsdosisformulierung für die orale Verabreichung verabreicht wird.

## Revendications

1. Composition comprenant une quantité thérapeutiquement active d'un corticostéroïde topique pour utilisation dans le traitement ou le soulagement des symptômes ou de l'inflammation associés à une maladie de reflux gastro-oesophagien (RGO) chez un individu, où le corticoïde topique est l'un des suivants : budésonide, fluticasone, mométasone furoate, ciclésonide, béclométhasone, désonide, ou un ester pharmaceutiquement acceptable de ceux-ci, ou une combinaison de ceux-ci, par administration topique à l'oesophage de l'individu.

2. Composition pour utilisation selon la revendication 1, où la maladie de reflux gastro-oesophagien est une maladie de reflux non érosif (NERD) ou une oesophagite érosive (EE).

3. Composition pour utilisation selon la revendication 1, où 100 µg/jour à 20 mg/jour du corticostéroïde sont administrés à l'individu.

4. Composition pour utilisation selon la revendication 3, où entre 250 µg/jour à 5 mg/jour du corticostéroïde sont administrés à l'individu.

5. Composition pour utilisation selon la revendication 1, où la maladie de reflux gastro-oesophagien (RGO) est réfractaire à un inhibiteur d'acide.

6. Composition pour utilisation selon la revendication 1, comprenant en outre l'administration d'une quantité thérapeutiquement active d'un H2RA, d'un inhibiteur de la pompe à protons (IPP), ou d'une combinaison de ceux-ci audit individu.

7. Composition pour utilisation selon la revendication 6, où le H2RA est choisi parmi les suivants : cimétidine, famotidine, nizatidine et ranitidine.

8. Composition pour utilisation selon la revendication 6, où le H2RA est administré à une teneur comprise entre 1 mg et 500 mg.

9. Composition pour utilisation selon la revendication 6, où l'inhibiteur de pompe à protons est choisi parmi les suivants : oméprazole, hydroxyoméprazole, ésoméprazole, ténatoprazole, lansoprazole, pantoprazole, rabeprazole, dontoprazole, habéprazole, perprazole, ransoprazole, pariprazole, léminoprazole, S-ténatoprazole-Na et dexlansoprazole.

10. Composition pour utilisation selon la revendication 6, où l'inhibiteur de la pompe à protons est administré à une teneur comprise entre environ 1 mg et 600 mg.

11. Composition pour utilisation selon la revendication 1, comprenant le corticoïde et au moins un excipient augmentant l'interaction du corticoïde avec l'oesophage de l'individu.

12. Composition pour utilisation selon la revendication 11, où la viscosité de la composition est comprise entre 90 mPa.s et 600 mPa.s, où la viscosité mesurée à 25 °C et une vitesse de cisaillement d'environ 13,2 s⁻¹.

13. Composition pour utilisation selon la revendication 1 ou 12, où l'excipient est un amplificateur de viscosité, un agent muco-adhésif, un agent amplifiant l'absorption ou l'une de leurs combinaisons.

14. Composition pour utilisation selon la revendication 11, où l'excipient est choisi parmi les suivants : acacia (gomme arabique), agar, silicate d'aluminium et de magnésium, alginate de sodium, stéarate de sodium, fucus vésiculeux, bentonite, carbomère, carraghénane, carbopol, cellulose, cellulose microcristalline (MCC), caroube, chondrus, dextrose, furcellarane, gélatine, gomme ghatti, gomme de guar, hectorite, lactose, sucrose, maltodextrine, mannitol, sorbitol, miel, amidon de maïs, amidon de blé, amidon de riz, amidon de pomme de terre, gélatine, gomme de sterculia, gomme xanthum, polyéthylène glycol (par exemple PEG 200-4500), gomme adragante, éthylcellulose, éthylhydroxyéthylcellulose, éthylméthylcellulose, méthylcellulose, hydroxyéthylcellulose, hydroxyéthylméthylcellulose, hydroxypropylcellulose, poly(méthacrylate d'hydroxyéthyle), oxypolygélatine, pectine, polygéline, povidone, carbonate de propylène, copolymère éther de méthyle et de vinyle/anhydride maléique (PVM/MA), poly(méthacrylate de méthoxyéthyle), poly(méthacrylate de méthoxyéthoxyéthyle), hydroxypropylcellulose, hydroxypropylméthyl-cellulose (HPMC), carboxymethyl-cellulose sodium (CMC), dioxyde de silicium, polyvinylpyrrolidone (PVP : povidone), un mélange de dextrose, de maltodextrine et de sucralose, et leurs combinaisons.

15. Composition pour utilisation selon la revendication 14, où l'excipient est une combinaison CMC/MCC présentant un rapport massique du mélange d'environ 11/89.

16. Composition pour utilisation selon la revendication 1, où le corticostéroïde est administré dans une formule de dose unitaire pour administration orale.
